# EUROPÄISCHE PATENTSCHRIFT

(11) **EP 1 102 767 B1**
(45) Veröffentlichungstag und Bekanntmachung des Hinweises auf die Patenterteilung: **02.11.2005**
(21) Anmeldenummer: 99938272.4
(22) Anmeldetag: 16.07.1999
(51) Int. Cl.: C07D 471/04, C07D 403/04, A61K 31/505

(54) **SUBSTITUIERTE PYRAZOLDERIVATE**
SUBSTITUTED PYRAZOLE DERIVATIVES
DERIVES DE PYRAZOLE SUBSTITUES

(30) Priorität: 29.07.1998 DE 19834047
(43) Veröffentlichungstag der Anmeldung: 30.05.2001
(73) Patentinhaber: Bayer HealthCare AG, 51368 Leverkusen (DE)
(72) Erfinder: STRAUB, Alexander, D-42113 Wuppertal (DE); FEURER, Achim, D-51519 Odenthal (DE); ALONSO-ALIJA, Cristina, D-42781 Haan (DE); STAHL, Elke, D-51467 Bergisch Gladbach (DE); STASCH, Johannes-Peter, D-42651 Solingen (DE); PERZBORN, Elisabeth, D-42327 Wuppertal (DE); HÜTTER, Joachim, D-42349 Wuppertal (DE); DEMBOWSKY, Klaus, D-69198 Schriesheim (DE)
(86) Internationale Anmeldenummer: PCT/EP1999/005073
(87) Internationale Veröffentlichungsnummer: WO 2000/006568

(56) Entgegenhaltungen:
- EP-A- 0 667 345
- WO-A-98/16507
- WO-A-98/23619
- CORSI, GIORGIO ET AL: "1-Halobenzyl-1H-indazole-3-carboxylic acids. A new class of antispermatogenic agents" J. MED. CHEM. (1976), 19(6), 778-83 , XP002125144

## Beschreibung

Die vorliegende Erfindung betrifft substituierte Pyrazolderivate, Verfahren zu ihrer Herstellung und ihre Verwendung als Arzneimittel, insbesondere als Arzneimittel zur Behandlung von Herz-Kreislauf-Erkrankungen.

Es ist bereits bekannt, daß 1-Benzyl-3-(substituierte heteroaryl)-kondensierte PyrazolDerivate die Tluombozytenaggregation inhibieren (vgl. EP 667 345 A1).

WO 98/16223 offenbart die Verwendung von 1-Benzyl-3-(substituiertes-Hetaryl)-kondensierten Pyrazolderivaten zur Behandlung von speziellen Erkrankungen des Herz-Kreislaufsystems und des Zentralnervensystems.

WO 98/16507 offenbart Heterocyclylmethyl-substituierte Pyrazolderivate und ihre Verwendung bei der Behandlung von Herz-Kreislauf-Erkrankungen.

WO 98/23619 offenbart ebenfalls substituierte Pyrazolderivate zur Behandlung von Herz-Kreislauf-Erkrankungen.

Die vorliegende Erfindung betrifft substituierte Pyrazolderivate der allgemeinen Formel (I), 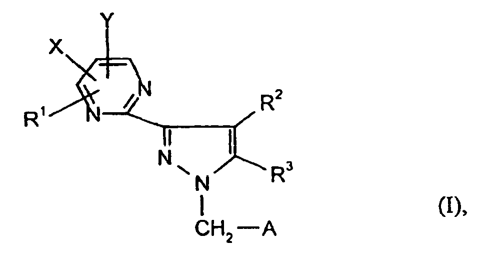 in welcher
R¹, X und Y an den dargestellten Positionen des Pyrimidinrings angeknüpft sind: 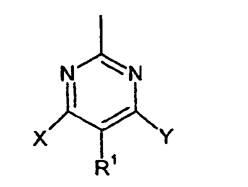
- R¹: für einen Cyclopropyl, Cyclobutyl, Cyclopentenyl, Cyclopentyl, Cyclohexyl, 1-Hydroxycyclopropyl oder 1-(Fluormethyl)cyclopropyl - Rest steht,
- X: für eine NH₂-Gruppe steht,
- Y: für Wasserstoff oder eine NH₂-Gruppe.
R² und R³ unter Einbezug der Doppelbindung für einen anellierten Pyridylring stehen so daß sich ein Pyrazolo[3,4-b]pyridin als Grundkörper ergibt.

A für einen in ortho-Position zur Methylenbrücke durch Fluor substituierten Phenylring steht
- R¹: steht besonders bevorzugt für Cyclopropyl.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Außerdem wurden Verfahren zur Herstellung der erfindungsgemäßen Verbindungen der allgemeinen Formel (I) gefunden, dadurch gekennzeichnet, daß man
[A] Verbindungen der allgemeinen Formel (II) 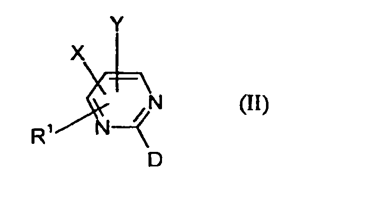 in welcher
   - R¹, X und Y: die oben angegebene Bedeutung haben,
   und
   - D: für Reste der Formeln 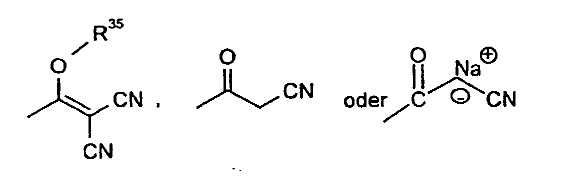
   steht,
   in welchen
   - R³⁵: für C₁-C₄-Alkyl steht,
   durch Umsetzung mit Verbindungen der allgemeinen Formel (III)

   A-CH₂-NH-NH₂ (III)

   in welcher
   - A: die oben angegebene Bedeutung hat
   in inerten Lösemitteln, gegebenenfalls in Anwesenheit einer Base, in die Verbindungen der allgemeinen Formel (IV) oder (IVa) 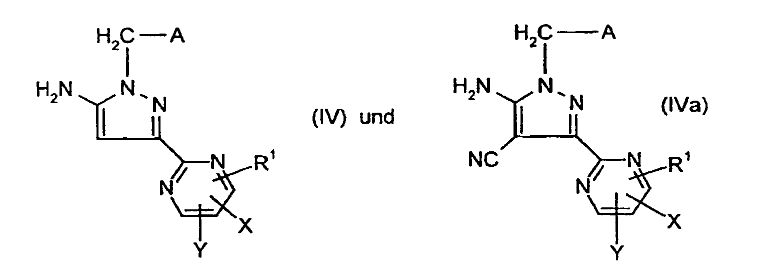 in welcher
   - A, X, Y und R¹: die oben angegebene Bedeutung haben,
   überführt,
   und im Fall der Verbindungen der allgemeinen Formel (IVa) anschließend mit Carbonsäuren, Nitrilen, Formamiden oder Guanidiniumsalzen cyclisiert,
   und im Fall der Verbindungen der allgemeinen Formel (IV) mit 1,3-Dicarbonyl-Derivaten, deren Salze, Tautomeren, Enolether oder Enaminen, in Anwesenheit von Säuren und gegebenenfalls unter Mikrowellen cyclisiert,
   oder
[B] Verbindungen der allgemeinen Formel (VII) 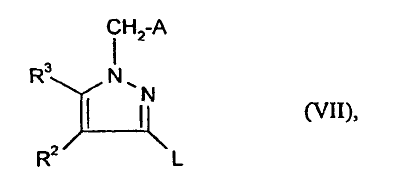 in welcher
   - A¹, R² und R³: die oben angegebene Bedeutung haben,
   und
   - L: für einen Rest der Formel -SnR³⁶R³⁷R³⁸, ZnR³⁹, Iod, Brom oder Triflat steht,
   worin
   R³⁶, R³⁷ und R³⁸ gleich oder verschieden sind und geradkettiges oder verzweigtes Alkyl mit bis zu 4 Kohlenstoffatomen bedeuten,
   und
   R³⁹ Halogen bedeutet, mit Verbindungen der allgemeinen Formel (VIII) 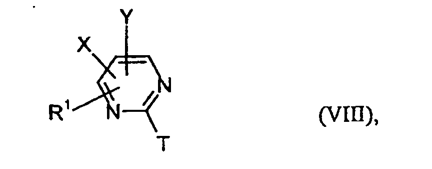 in welcher
   X, Y und R¹ die oben angegebene Bedeutung haben
   und
   im Fall L = SnR³⁶R³⁷R³⁸ oder ZnR³⁹
   T für Triflat oder für Halogen, vorzugsweise für Brom steht,
   und
   im Fall L = Iod, Brom oder Triflat
   T für einen Rest der Formel SnR^{36'}R^{37'}TR^{38'}, ZnR^{39'} oder BR⁴⁰R⁴¹ steht,
   worin
   R^{36'}, R^{37'}, R^{38'} und R^{39'} die oben angebene Bedeutung von R³⁶, R³⁷, R³⁸ und R³⁹ haben und mit diesen gleich oder verschieden sind,
   R⁴⁰ und R⁴¹ gleich oder verschieden sind und Hydroxy, Aryloxy mit 6 bis 10 Kohlenstoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden, in einer palladiumkatalysierten Reaktion in inerten Lösemitteln umsetzt, gegebenenfalls in Gegenwart einer Base,
   oder
[C] Amidine der allgemeinen Formel (IX) 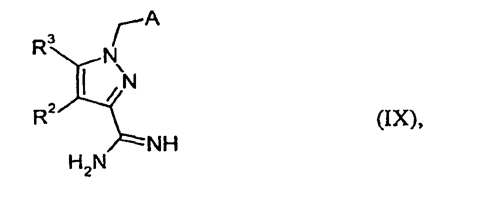 in welcher
   - A, R² und R³: die oben angegebene Bedeutung haben,
   beispielsweise mit Verbindungen der allgemeinen Formel (X), (Xa), (Xb) oder (Xc) 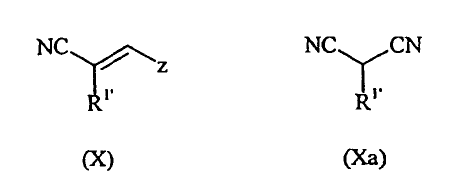 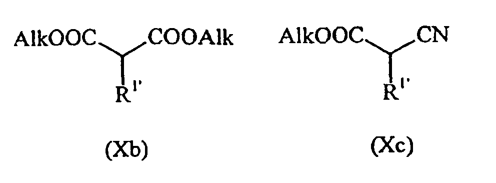 in welchen
   - R^{1'}: für den oben unter R¹ aufgeführten gegebenenfalls substituierten Cycloalkylrest steht;
   - Alk: für geradkettiges oder verzweigtes Alkyl mit bis zu 8 Kohlenstoffatomen, vorzugsweise bis zu vier Kohlenstoffatomen steht;
   und
   - Z: für eine NH₂-Gruppe, Monoalkylaminogruppe mit bis zu 7 Kohlenstoffatomen, Dialkylaminogruppe mit bis zu 7 Kohlenstoffatomen, einen über den Stickstoff gebundenen Piperidino- oder Morpholinorest, Hydroxyl, Alkoxy mit bis zu 7 Kohlenstoffatomen, Acyloxy mit bis zu 7 Kohlenstoffatomen oder Aroyloxy mit 6 bis 10 Kohlenstoffatomen steht,
   umsetzt,
und gegebenenfalls die unter X, Y, R¹, R², R³ und/oder A aufgeführten Substituenten nach üblichen Methoden, vorzugsweise durch Acylierung und Derivatisierung freier Aminogruppen, Chlorierung, katalytische Hydrierung, Reduktion, Oxidation, Abspaltung von Schutzgruppen und/oder nucleophiler Substitution variiert oder einführt.

Die unter R² und R³ aufgeführten Heterocyclen können auch durch Umsetzung der entsprechend substituierten Verbindungen der allgemeinen Formel (II) nach anderen bekannten heterocyclischen Synthesen eingeführt werden.

Das erfindungsgemäße Verfahren [C] kann durch folgendes Ausführungsbeispiel erläutert werden:

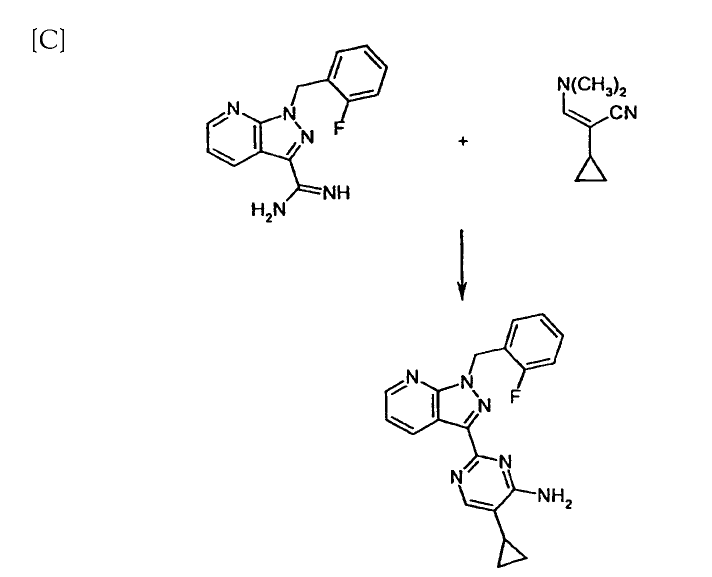

Als Lösemittel für die einzelnen Schritte des Verfahrens [A] eignen sich hierbei inerte organische Lösemittel, die sich unter den Reaktionsbedingungen nicht verändern. Hierzu gehören Ether, wie Diethylether oder Tetrahydrofuran, DME, Dioxan, Alkohole wie Methanol und Ethanol, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt ist Tetrahydrofuran, Dimethylformamid, Toluol, Dioxan oder Dimethoxyethan.

Als Basen können bei den erfindungsgemäßen Verfahren im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)-amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat, Triethylamin und Natriumhydrid.

Die Base wird bei der Umsetzung der Verbindungen der Formel (II) mit den Verbindungen der Formel (III) in einer Menge von 1 mol bis 5 mol, bevorzugt von 1 mol bis 3 mol, bezogen auf 1 mol der Verbindung der allgemeinen Formel (II) eingesetzt.

Die Umsetzung der Verbindungen der Formel (II) mit den Verbindungen der Formel (III) wird im allgemeinen in einem Temperaturbereich von 0°C bis 150°C, bevorzugt von +20°C bis +110°C durchgeführt.

Diese Umsetzung kann bei normalen, erhöhtem oder bei erniedrigtem Druck durchgeführt werden (z.B. 0,5 bis 5 bar). Im allgemeinen arbeitet man bei Normaldruck.

Als Säuren für die gegebenenfalls bei den erfindungsgemäßen Verfahren durchzuführenden Cyclisierungsreaktionen eignen sich im allgemeinen Protonensäuren. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure.

Die gegebenenfalls innerhalb der erfindungsgemäßen Verfahren durchzuführenden katalytischen Hydrierungsreaktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Die gegebenenfalls innerhalb der erfindungsgemäßen Verfahren durchzuführenden Chlorierungsreaktionen erfolgen im allgemeinen mit den üblichen Chlorierungsmitteln wie beispielsweise PCl₃, PCl₅, POCl₃ oder elementarem Chlor. Bevorzugt ist im Rahmen der Erfindung POCl₃.

Die gegebenenfalls innerhalb der erfindungsgemäßen Verfahren durchzuführenden Acylierungen und Derivatisierungen freier Aminogruppen können nach üblichen, dem Fachmann geläufigen Methoden durchgeführt werden. Beispielsweise können entsprechende freie Aminogruppen durch Umsetzung mit einem Säurehalogenid, vorzugsweise einem Säurechlorid, oder mit einem Säureanhydrid, in Gegenwart einer Base wie beispielsweise Natriumhydrid, Pyridin oder Dimethylaminopyridin in einem Lösungsmittel wie Tetrahydrofuran oder Dichlormethan in die jeweiligen Amide, durch Reaktion mit einem entsprechenden Aldehyd in einem Lösungsmittel wie Ethanol oder Acetonitril in die jeweiligen N,O-Halbacetale oder N,O-Vollacetale, durch Umsetzung mit einem Sulfonylhalogenid, vorzugsweise einem Sulfonylchlorid, in die jeweiligen Sulfonamide, durch Reaktion mit einem Chlorameisensäureester in die jeweiligen Urethane oder durch Umsetzung mit einem Isocyanat in einem Lösungsmittel wie Dichlormethan in die jeweiligen Hamstoffderivate überführt werden.

Die gegebenenfalls innerhalb der erfindungsgemäßen Verfahren durchzuführenden nucleophilen Substitutionen und Vilsmeierreaktionen werden nach üblichen, dem Fachmann geläufigen Methoden durchgeführt.

Die gegebenenfalls innerhalb der erfindungsgemäßen Verfahren durchzuführenden Reduktionen werden im allgemeinen mit Reduktionsmitteln, bevorzugt mit solchen, die für die Reduktion von Carbonyl zu Hydroxyverbindungen geeignet sind, durchgeführt. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithiumtrialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird hierbei im allgemeinen in einer Menge von 1 mol bis 6 mol, bevorzugt von 1 mol bis 4 mol bezogen auf 1 mol der zu reduzierenden Verbindungen, eingesetzt.

Die gegebenenfalls innerhalb der erfindungsgemäßen Verfahren durchzuführenden Reduktionen werden im allgemeinen in einem Temperaturbereich von -78°C bis +50°C durchgeführt, bevorzugt von -78°C bis 0°C im Falle des DIBAH und 0°C bis Raumtemperatur im Falle des NaBH₄.

Die gegebenenfalls innerhalb der erfindungsgemäßen Verfahren durchzuführenden Reduktionen werden im allgemeinen bei Normaldruck durchgeführt. Es ist aber auch möglich, bei erhöhtem oder erniedrigtem Druck zu arbeiten.

Die Verbindungen der allgemeinen Formeln (II) und (III) sind an sich bekannt oder nach üblichen Methoden herstellbar (vgl. hierzu: J. Hromatha et al., Monatsh. Chem. 1976, 107, 233).

Die Verbindungen der allgemeinen Formeln (IV) und (IVa) sind teilweise bekannt und können wie oben beschrieben hergestellt werden.

Als Lösemittel für das Verfahren [B] eignen sich inerte organische Lösemittel, die sich unter den Reaktionsbedingungen nicht verändern. Hierzu gehören Ether, wie Diethylether oder Tetrahydrofuran, DME, Dioxan, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt ist Tetrahydrofuran, Dimethylformamid, Toluol, Dioxan oder Dimethoxyethan.

Die Umsetzung der Verbindungen der Formel (VII) mit den Verbindungen der Formel (VIII) wird im allgemeinen in einem Temperaturbereich von 0°C bis 150°C, bevorzugt von +20°C bis +110°C durchgeführt.

Diese Umsetzung kann bei normalen, erhöhtem oder bei erniedrigtem Druck durchgeführt werden (z.B. 0,5 bis 5 bar). Im allgemeinen arbeitet man bei Normaldruck.

Als Palladiumverbindungen im Rahmen der vorliegenden Erfindung eignen sich im allgemeinen PdCl₂(P(C₆H₅)₃)₂, Palladium-bis-dibenzylidenaceton (Pd(dba)₂), [1,1'-Bis-(diphenylphosphino)ferrocen]-Palladium(II)-chlorid (Pd(dppf)Cl₂) oder Pd(P(C₆H₅)₃)₄, Bevorzugt ist Pd(P(C₆H₅)₃)₄.

Die Verbindungen der allgemeinen Formel (VII) sind an sich bekannt oder nach üblichen Methoden herstellbar (vgl. beispielsweise K. Kirschke in: Houben-Weyl, Methoden der organischen Chemie, Thieme-Verlag Stuttgart, 4. Aufl., Band E8b, Teil 2, 399-763; insbesondere bezüglich Pyrazolopyridinen: C.R. Hardy in A.R. Katritzky (Hrsg.), Adv. Het. Chem. 1984, 36, 343-409; insbesondere bezüglich Pyrazolopyrimidinen: M.H. Elgnadi et al., Adv. Het. Chem. 1987, 41, 319-376). Die Herstellung der entsprechenden Halogenpyrazolo[3,4-b]pyrimidine und zinnorganischen Pyrazolo[3,4-b]pyrimidine der Formel (VII) ist in der WO 98/23619 beschrieben und kann analog auch für die entsprechenden Triflat- und zinkorganischen Verbindungen der Formel (VII) durchgeführt werden.

Die Verbindungen der allgemeinen Formel (VIII) sind bekannt und nach üblichen Methoden herstellbar (vgl. beispielsweise M.G. Hoffmann et al. in: Houben-Weyl, Methoden der organischen Chemie, 4.Aufl., Band E9b, Teil 1, S. 1-249; A. Weissenberger et al., The Chemistry of heterocyclic compounds - Pyrimidines, 1962, 16; ibid 1970, 16, Suppl. 1, ibid 1985, 16, Suppl. 2; ibid 1994, 52).

Das Verfahren [C] wird in einem Temperaturbereich von 80°C bis 120°C, vorzugsweise bei 100°C bis 110°C oder unter Rückfluß durchgeführt.

Als Lösemittel können beispielsweise die Reagentien der allgemeinen Formel (X), (Xa), (Xb) oder (Xc) fungieren. Die Reaktion kann aber auch in anderen geeigneten Lösemitteln durchgeführt werden, wie z.B. Toluol, Methanol oder Dichlormethan. Leichtsiedende Lösungsmittel wie z.B. Dichlormethan können im Laufe der Reaktion abdestilliert werden.

Das Verfahren [C] kann bei normalen, erhöhtem oder bei erniedrigtem Druck durchgeführt werden (z.B. 0,5 bis 5 bar). Im allgemeinen arbeitet man bei Normaldruck.

Die Amidine der allgemeinen Formel (IX) sind neu und daher ein weiterer Gegenstand der Erfindung. Sie können hergestellt werden, indem man die Verbindungen der allgemeinen Formel (XI) 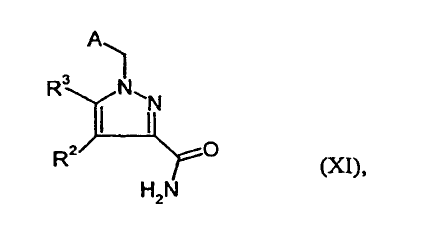 in welcher
- A, R² und R³: die oben angegebene Bedeutung haben,
zunächst in Ethern mit Trifluoressigsäureanhydrid (TFAA) und in Anwesenheit von Basen zu der Verbindung der allgemeinen Formel (XII) 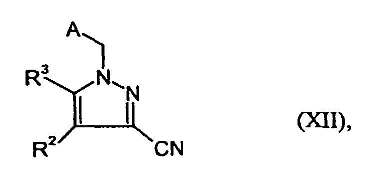 in welcher
- A, R² und R³: die oben angegebene Bedeutung haben,
umsetzt,
anschließend mit Natriummethanolat die Verbindungen der allgemeinen Formel (XIII) 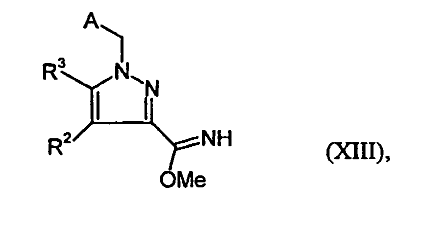 in welcher
- A, R² und R³: die oben angegebene Bedeutung haben,
herstellt, in einem nächsten Schritt durch Umsetzung mit NH₄Cl und Eisessig in Alkoholen in das entsprechende Amidin HCl-Salz der allgemeinen Formel (XIV) 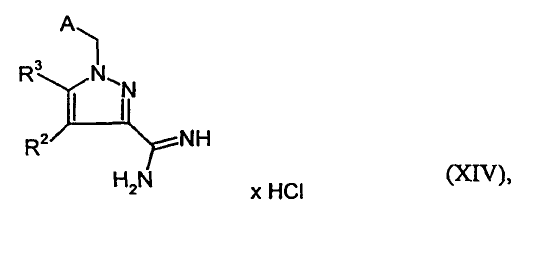 in welcher
- A, R² und R³: die oben angegebene Bedeutung haben,
überführt und in einem letzten Schritt mit Basen, vorzugsweise Natriumcarbonat oder Alkalialkoholat wie Natriumethanolat umsetzt.

Als Lösemittel für die Umsetzung der Verbindungen der allgemeinen Formel (XI) zu den Verbindungen der Formel (XII) eignen sich Ether, wie Diethylether oder Tetrahydrofuran, Dimethylformamid und Dioxan; bevorzugt ist Tetrahydrofuran.

Als Basen können hierbei organische Amine (Trialkyl(C₁-C₆)-amine) wie Triethylamin, oder Heterocyclen wie 1,4-Diazabicyclo[2.2.2]octan (DABCO), 1,8-Diazabicyclo[5.4.0]undec-7-en (DBU), Pyridin, Dimethylaminopyridin, Methylpiperidin oder Morpholin eingesetzt werden. Bevorzugt ist Pyridin.

Die Umsetzung erfolgt in einem Temperaturbereich von 0°C bis 40°C, vorzugsweise bei Raumtemperatur.

Die Umsetzung kann bei normalen, erhöhtem oder bei erniedrigtem Druck durchgeführt werden (z.B. 0,5 bis 5 bar). Im allgemeinen arbeitet man bei Normaldruck.

Das Amid (XI) kann beispielsweise durch Verseifung eines entsprechenden Esters als Ausgangsverbindung mit einer Base zur Säure, deren Überführung in das Säurechlorid nach üblichen Methoden z.B. mittels SOCl₂ oder POCl₃ und anschließender Umsetzung mit Ammoniak hergestellt werden.

Die Eliminierung von Wasser aus dem Amid (XI) zum Nitril (XII) kann mit allen üblichen wasserentziehenden Mitteln durchgeführt werden. Erfindungsgemäß bevorzugt ist Trifluoressigsäureanhydrid (TFAA).

Die Überführung des Nitrils der Formel (XII) in den Iminoether der Formel (XIII) kann sowohl im Sauren, wie z.B. mit HCl/Alkohol-Gemischen, als auch im Basischen wie z.B. mit Methanol/Natriummethanolat erfolgen. Sie erfolgt üblicherweise bei 0°C bis 40°C, beispielsweise bei Raumtemperatur.

Als Lösemittel für Umsetzung der Verbindungen der allgemeinen Formel (XIII) zu den Verbindungen der Formel (XIV) eignen sich Alkohole wie Methanol oder Ethanol. Bevorzugt ist Methanol.

Die Umsetzung erfolgt in einem Temperaturbereich von 0°C bis 40°C, vorzugsweise bei Raumtemperatur.

Die Umsetzung kann bei normalem, erhöhtem oder bei erniedrigtem Druck durchgeführt werden (z.B. 0,5 bis 5 bar). Im allgemeinen arbeitet man bei Normaldruck.

Als Basen für die Freisetzung der Verbindungen der allgemeinen Formel (IX) aus Verbindungen der allgemeinen Formel (XIV) eignen sich anorganische oder organische Basen. Hierzu gehören beispielsweise Alkalihydroxide wie Natriumhydroxid oder Kaliumhydroxid, Erdalkalihydroxide wie Bariumhydroxid, Alkalicarbonate wie Natriumcarbonat oder Kaliumcarbonat, Erdalkalicarbonate wie Calciumcarbonat und Alkalialkoholate wie Natriummethanolat. Bevorzugt sind Natriumcarbonat und Natriummethanolat.

Die Darstellung des Pyrimidinrings erfolgt nach den üblichen Methoden (vgl. beispielsweise M.G. Hoffmann et al. in: Houben-Weyl, Methoden der organischen Chemie, 4.Aufl., Band E9b, Teil 1, S. 1-249; A. Weissenberger et al., The Chemistry of heterocyclic compounds - Pyrimidines, 1962, 16; ibid 1970, 16, Suppl. 1, ibid 1985, 16, Suppl. 2; ibid 1994, 52).

Hierbei kann man von den Iminoethern der Formel (XIII) ausgehen und diesen z.B. mit einem geeigneten Enamin umsetzen. Man kann aber auch den Iminoether zunächst mittels Ammoniak oder dessen Salzen in ein entsprechendes Amidin überführen und dieses entweder als freie Base (IX) oder als Salz (XIV) mit Enaminen, Acetalen, Enolethern, Aldehyden, Enolaten, Malonitrilestem oder Malondinitrilen umsetzen.

Die bei dieser Umsetzung gegebenenfalls einzusetzenden Enamine können z.B. aus C-H-aciden Verbindungen wie Acetonitrilderivaten nach bekannten Methoden durch Umsetzung mit Dimethylformamid-Derivaten wie z.B. Bis(dimethylamino)-tert-butoxymethan, Dialkoxy-dialkylamino-methanen hergestellt werden.

Die Verbindungen der allgemeinen Formel (XI) sind neu und daher ein weiterer Gegenstand der Erfindung. Sie können hergestellt werden, indem man die Verbindungen der allgemeinen Formel (XV) 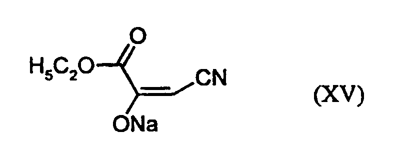 mit den Verbindungen der allgemeinen Formel (XVI)

A―CH₂-NH-NH₂ (XVI)

in Ethern, vorzugsweise Dioxan und Trifluoressigsäure in die Verbindungen der allgemeinen Formel (XVII) 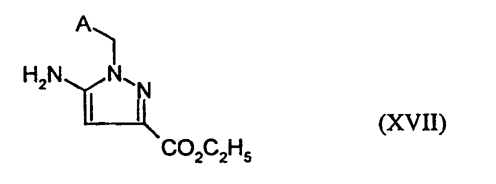 überführt,
anschließend durch Umsetzung mit den Verbindungen der allgemeinen Formel (XVIII)

Z―CH=CH―CHO (XVIII)

worin
- Z: die oben angegebenen Bedeutungen, insbesondere -N(CH₃)₂, haben kann,
in inerten Lösemitteln, vorzugsweise Dioxan, die Verbindungen der allgemeinen Formel (XIX) 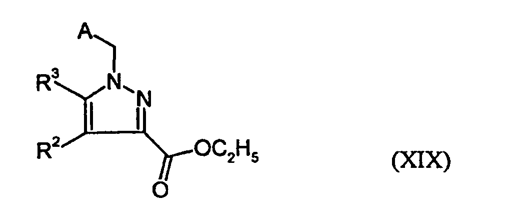 herstellt und in einem letzten Schritt mit Ammoniak in Methanol versetzt.

Anstelle des Natriumsalzes des Enolates (XV) können auch Enolether, Ketone oder Enamine eingesetzt werden.

Gegebenenfalls kann die Umsetzung der Verbindungen der allgemeinen Formeln (XV) und (XVI) zu (XVII) auch über Zwischenverbindungen der Formeln (A) und (B), 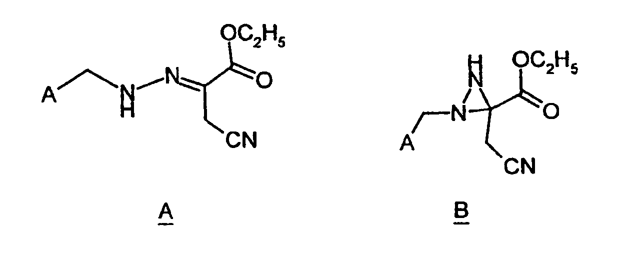 bei Raumtemperatur erfolgen, die ebenfalls neu sind. Diese stellen daher einen weiteren Gegenstand der Erfindung dar.

Die Verbindungen der allgemeinen Formel (X) sind neu und können beispielsweise hergestellt werden, indem man die Verbindungen der Formel (XX) oder (XXa)

[Alk₂N]₂―CH―OAlk' (XX)

Alk₂N―CH―[OAlk']₂ (XXa)

worin
- Alk und Alk': gleich oder verschieden sind und für geradkettiges oder verzweigtes Alkyl mit bis zu 5 Kohlenstoffatomen stehen,
mit Verbindungen der Formel (XXI)

R^{1'}-CH₂-CN (XXI)

worin
- R^{1'}: für den oben unter R¹ aufgeführten Cycloalkylrest steht
umsetzt.

Die Verbindungen der allgemeinen Formeln (XX), (XXa) und (XXI) sind bekannt oder nach üblichen Methoden herstellbar.

Die Verbindungen der allgemeinen Formeln (XII), (XIII), (XIV), (XV), (XVII), (XVIII) und (XIX) sind teilweise neu und können wie oben beschrieben hergestellt werden.

Der Pyrimidinrest kann auch mit Hilfe des Reagenz der Formel (Xa) aufgebaut werden, das beispielsweise folgendermaßen zugänglich ist:

### Verbindungen der allgemeinen Formel

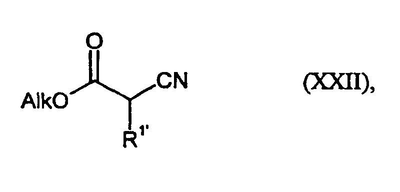

worin
- R^{1'}: die oben angegebene Bedeutung hat und Alk für einen Alkylrest mit bis zu 4 Kohlenstoffatomen steht,
werden mittels Ammoniak in geeigneten Lösungsmitteln, vorzugsweise Alkoholen wie Methanol, bei Temperaturen von 0°C bis 40°C, vorzugsweise Raumtemperatur, in Verbindungen der allgemeinen Formel (XXIII) 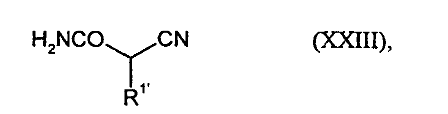 überführt, worin R^{1'} die oben angegebene Bedeutung hat,
und diese anschließend nach übliche Methoden durch wasserentziehende Mittel, wie beispielsweise das Burgess-Reagenz, POCl₃, P₂O₅, SOCl₂, Trifluoressigsäureanhydrid/Pyridin, umgesetzt.

Bei Verwendung des Burgess-Reagenz wird die Reaktion vorzugsweise in inerten Lösemitteln wie Ethern oder chlorierten Kohlenwasserstoffen durchgeführt. Als Beispiele seien Dichlormethan und Tetrahydrofuran genannt. Vorzugsweise wird ein 1:2-Gemisch der vorgenannten Lösemittel verwendet. Die Reaktion wird bei Temperaturen von 0°C bis 40°C, vorzugsweise bei Raumtemperatur, durchgeführt.

Die Verbindungen der Formel (XXII) sind bekannt und/oder auf einfache und dem Fachmann geläufige Weise zugänglich.

Die Verbindungen der Formel (X) unterliegen zum Teil der Keto-Enol-Tautomerie, z.B.:

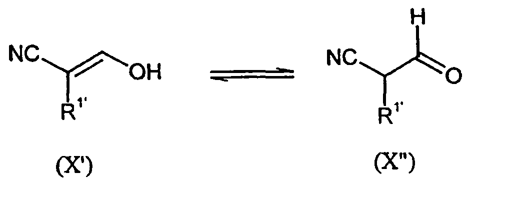

Im Fall, daß R² und R³ unter Einbezug der Doppelbindung einen Phenylring bilden, werden die entsprechenden 3-Cyan-Indazole mit Verbindungen der allgemeinen Formel (XXIV) 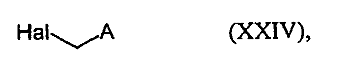 in welcher
- A: die oben angegebene Bedeutung hat,
und
- Hal: für Cl, Br oder I, vorzugsweise Br, steht,
in inerten Lösemitteln, vorzugsweise mit Tetrahydrofuran in Anwesenheit einer Base, vorzugsweise Natriumhydrid zu den Verbindungen der allgemeinen Formel (XXV) 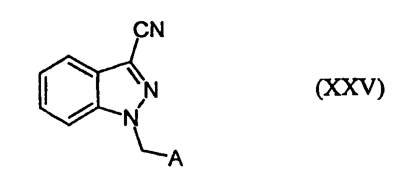 in welcher
- A: die oben angegebene Bedeutung hat,
umgesetzt und abschließend mit Ammoniumchlorid und Natriummethanolat wie oben beschrieben versetzt.

Die Verbindungen der allgemeinen Formel (XXIV) sind bekannt oder nach üblichen Methoden herstellbar.

Die Verbindungen der allgemeinen Formel (XXV) sind neu und können wie oben beschrieben hergestellt werden.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen der allgemeinen Formel (I) führen zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen der allgemeinen Formel (I) die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate. Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektile Dysfunktion, weibliche sexuelle Dysfunktion und Inkontinenz eingesetzt werden.

Die in der vorliegenden Erfindung beschriebenen Verbindungen der allgemeinen Formel (I) stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Him-Traumas. Ebenso können die erfindungsgemäßen Verbindungen der allgemeinen Formel (I) zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Darüber hinaus umfaßt die Erfindung die Kombination der erfindungsgemäßen Verbindungen der allgemeinen Formel (I) mit organischen Nitraten und NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Bevorzugt sind Natriumnitroprussid, Nitroglycerin, Isosorbiddinitrat, Isosorbidmononitrat, Molsidomin und SIN-1.

Außerdem umfaßt die Erfindung die Kombination mit Verbindungen, die den Abbau von cyclischem Guanosinmonophosphat (cGMP) inhibieren. Dies sind insbesondere Inhibitoren der Phosphodiesterasen 1, 2 und 5; Nomenklatur nach Beavo und Reifsnyder (1990) TiPS 11 S. 150 bis 155. Durch diese Inhibitoren wird die Wirkung der erfindungsgemäßen Verbindung potenziert und der gewünschte pharmakologische Effekt gesteigert.

Zur Feststellung der kardiovaskulären Wirkungen wurden folgende Untersuchungen durchgeführt: In in vitro-Untersuchungen isolierten Enzym und an Zellen vaskulären Ursprungs wurde der Einfluß auf die Guanylatzyklase-abhängige cGMP-Bildung mit und ohne NO-Donor geprüft. Die antiaggregatorischen Eigenschaften wurden an mit Kollagen stimulierten menschlichen Thrombozyten gezeigt. Die gefäßrelaxierende Wirkung wurde an mit Phenylephrin vorkontrahierten Kaninchenaortenringen bestimmt. Die blutdrucksenkenden Wirkungen wurden an narkotisierten und wachen Ratten untersucht.

### Stimulation der rekombinanten löslichen Guanylatzyklase in vitro

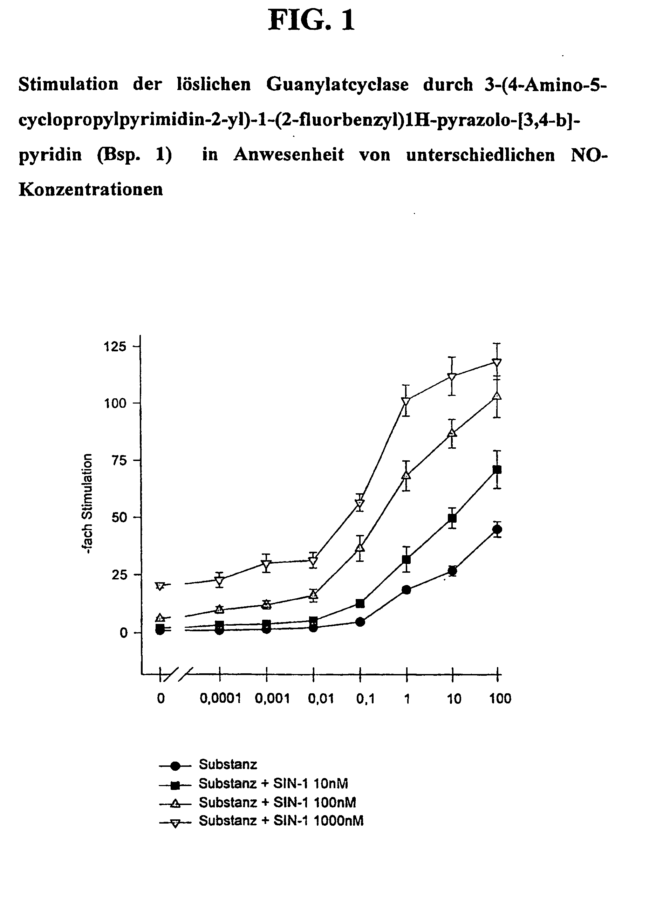

Die Untersuchungen zur Stimulation der rekombinanten löslichen Guanylatzyklase durch die erfindungsgemäßen Verbindungen mit und ohne NO-Donor wurden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch: Purified soluble guanylyl cyclase expressed in a baculovirus/Sf9 system: stimulation by YC-1, nitric oxide, and carbon oxide. J. Mol. Med. 77: 14-23 (1999). Die Ergebnisse sind in Fig. 1 gezeigt.

### Stimulation der löslichen Guanylatzyklase in primären Endothelzellen

Primäre Endothelzellen wurden aus Schweineaorten durch Behandlung mit Kollagenase-Lsg. isoliert. Anschließend wurden die Zellen in Kulturmedium bei 37°C /5% CO₂ bis zum Erreichen der Konfluenz kultiviert. Für die Untersuchungen wurden die Zellen passagiert, in 24-Loch Zellkulturplatten ausgesät und bis zum Erreichen der Konfluenz subkultiviert (~ 2 x 10⁵ Zellen / Vertiefung). Zur Stimulation der endothelialen Guanylatzyklase wurde das Kulturmedium abgesaugt und die Zellen einmal mit Ringerlösung gewaschen. Nach Entfernen der Ringerlösung wurden die Zellen in Stimulationspuffer 10 Minuten bei 37°C / 5% CO₂ inkubiert. Im Anschluß daran wurden die Testsubstanzen (Endkonzentration 1 µM) zu den Zellen pipettiert. Nach weiteren 10 Minuten wurde die Pufferlösung abgesaugt und 4°C kalter Stoppuffer zu den Zellen gegeben. Die Zellen wurden dann 16 Stunden lang bei -20°C lysiert. Anschließend wurden die das intrazelluläre cGMP enthaltenden Überstände abgenommen und die cGMP-Konzentrationen durch das cGMP-SPA-System (Amersham Buchler, Braunschweig) bestimmt. Die Ergebnisse sind in Tabelle 1 aufgeführt.

**Tabelle 1**

| Bsp. Nr. | Konzentration (µM) | 0 | 0.1 | 0.3 | 1.0 | 3.0 | 10 |
|---|---|---|---|---|---|---|---|
| 1 | cGMP (pmol/well) | 1.4 | 2.2 | 4.0 | 6.9 | 8.5 | 14.6 |

### Gefäßrelaxierende Wirkung in vitro

Kaninchen werden durch Nackenschlag betäubt und entblutet. Die Aorta wird entnommen, von anhaftendem Gewebe befreit, in 1,5 mm breite Ringe geteilt und einzeln unter einer Vorspannung in 5 ml-Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O; 1,4; KH₂PO₄: 1,2; NaHCO₃:25; Glucose: 10. Die Kontraktionskraft wird mit Statham UC2-Zellen erfaßt, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung untersucht und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀). Das Standardapplikationsvolumen beträgt 5 µl, der DMSO-Anteil in der Badlösung entspricht 0,1 %. Die Ergebnisse sind in Tabelle 2aufgeführt.

**Tabelle 2**

| Beispiel Nr. | IC₅₀ |
|---|---|
| 1 | 0.2 µM |

### Blutdruckmessungen an narkotisierten Ratten

Männliche Wistar-Ratten mit einem Körpergewicht von 300 - 350 g werden mit Thiopental (100 mg/kg i.p.) anästhesiert. Nach Tracheotomie wird in die Femoralarterie ein Katheter zur Blutdruckmessung eingeführt. Die zu prüfenden Substanzen werden in Transcutol, Cremophor EL, H₂O (10%/20%/70%) in einem Volumen von 1 ml/kg oral verabreicht. Die Ergebnisse sind in der nachstehenden Tabelle 3 aufgeführt.

**Tabelle 3**

| Bsp.-Nr. | Dosis (mg/kg/p.o.) | Max. Blutdrucksenkung (mm Hg) | Zeit (min) |
|---|---|---|---|
| 1 | 1 | 23 | 20 |
| 1 | 3 | 37 | 40 |

### Wirkung auf den mittleren Blutdruck von wachen, spontan hypertensiven Ratten

Kontinuierliche Blutdruckmessungen über 24 Stunden wurden an spontan hypertonen 200-250g schweren sich frei bewegenden weiblichen Ratten (MOL:SPRD) durchgeführt. Dazu waren den Tieren chronisch Druckaufnehmer (Data Sciences Inc., St. Paul, MN, USA) in die absteigende Bauchaorta unterhalb der Nierenarterie implantiert und der damit verbundene Sender in der Bauchhöhle fixiert worden.

Die Tiere wurden einzeln in Type III Käfigen, die auf den individuellen Empfängerstationen positioniert waren, gehalten und waren an einem 12-Stunden Hell/Dunkel-Rhythmus angepaßt. Wasser und Futter standen frei zur Verfügung.

Zur Datenerfassung wurde der Blutdruck jeder Ratte alle 5 Minuten für 10 Sekunden registriert. Die Meßpunkte wurden jeweils für eine Periode von 15 Minuten zusammengefaßt und der Mittelwert aus diesen Werten berechnet.

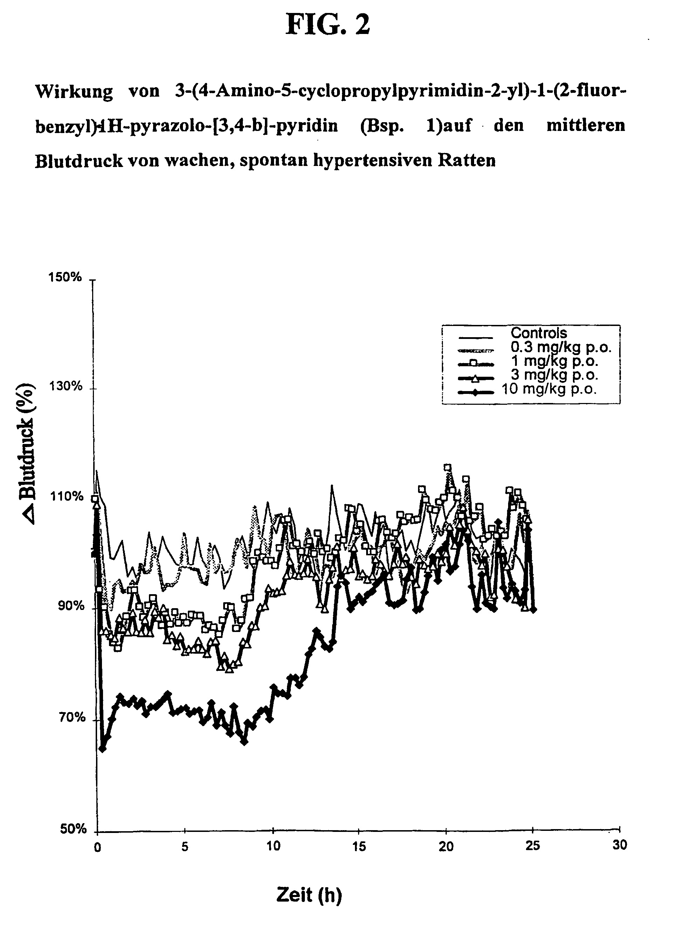

Die Prüfverbindungen wurden in einer Mischung aus Transcutol (10%), Cremophor (20%), H₂O (70%) gelöst und mittels Schlundsonde in einem Volumen von 2 ml/kg Körpergewicht oral verabreicht. Die Prüfdosen lagen zwischen 0,3 -30 mg/kg Körpergewicht. Die Ergebnisse sind in Fig. 2 gezeigt.

### Thrombozytenaggregationshemmung in vitro

Zur Bestimmung der Thrombozytenaggregation wurde Blut von gesunden Probanden beiderlei Geschlechts verwendet. Als Antikoagulans wurde einem Teil 3,8%iger Natriumzitratlösung 9 Teile Blut zugemischt. Das Blut wurde mit 900U/min für 20min zentrifugiert. Der pH Wert des gewonnenen plättchenreichen Plasmas wurde mit ACD-Lösung (Natriumcitrat/Citronensäure/Glucose) auf pH 6,5 eingestellt. Die Thrombozyten wurden anschließend abzentrifugiert und in Puffer aufgenommen und wiederum abzentrifugiert. Der Thrombozytenniederschlag wurde in Puffer aufgenommen und zusätzlich mit 2 mmol/l CaCl₂ versetzt.

Für die Aggregationsmessungen wurden Aliquots der Thrombozytensuspension mit der Prüfsubstanz 10 min bei 37°C inkubiert. Anschließend wurde die Aggregation durch Zugabe von Kollagen in einem Aggregometer ausgelöst und mittels der turbidometrischen Methode nach Born (Born, G.V.R., J.Physiol. (London), 168, 178-195, 1963) bei 37°C bestimmt. Die Ergebnisse sind nachstehend in Tabelle 2 aufgeführt.

**Tabelle 4**

| Bsp.-Nr. | IC₅₀ (µM) |
|---|---|
| 1 | 0,003 |

### Messung der erektionsfördernden Wirksamkeit von Guanylatcyclase-Stimulatoren

Für das Zustandekommen einer vollständigen und anhaltenden Erektion müssen die cavernösen Arterien und die gesamte Schwellkörperarchitektur, die aus einem Netzwerk von glatten Muskelzellen und kollagenem Bindegewebe gebildet wird, maximal dilatieren, damit sich der Corpus cavernosum vollständig mit Blut füllen kann (Anderson K.-E. and Wagner G.,"Physiology of Penile Erection.". *Physiological Reviews 75, 191-236 (1995);* Meinhardt W. Kropmann RF, Vermeig P, Lycclama a Nigelholt and Zwartendijk J. "The Influence of Medication on Erectile dysfunction." *Int. J. of Impotence Res. 9, 17-26 (1997).* Die Relaxation der glatten Muskulatur wird durch NO vermittelt, das bei sexueller Stimulation von nicht adreneregen, nicht cholinergen Nervenfasern in den Endothelzellen der Blutgefäße des Corpus cavernosum freigesetzt wird. NO aktiviert die Guanylatcyclase, der daraus resultierende Anstieg des cGMP führt zur Dilatation der glatten Muskulatur des Corpus cavernosum und damit zu einer Erektion. Zur Prüfung der Wirksamkeit der erfmdungsgemäßen Substanzen wurden wache Kaninchen eingesetzt. Die Spezies Kaninchen wurde gewählt, da die Neurophysiologie, die Haemodynamik und die Steuerung der Kontraktion und der Relaxation der glatten Muskulatur des Schwellkörpers bei Kanichen und Mensch recht ähnlich sind (Meyer MF, Taher H., Krah H., Staubesand J., Becker AJ, Kircher M., Mayer B., Jonas U., Forsmann WG., Stief Ch.G. "Intracarvenous Application of SIN-1 in Rabbit and Man: Functional and Toxcological Results." *Annals Urol. 27, 179-182 (1993);* Taub HC, Lerner,SE, Melman A, Christ GJ "Relationship between contraction and relaxation in human and rabbit corpus cavemosum." *Uralogy 42, 698-671, (1993)*.

### Methode:

Adulte, männliche Chinchilla-Kaninchen mit einem Gewicht von 3 -5 kg werden nach Lieferung mehrere Tage in Einzelhaltung adaptiert. Sie haben freien Zugang zu Wasser und können zwei Stunden pro Tag Futter zu sich nehmen. Die Tiere werden in einem 10/14 Stunden Tag-Nacht Rhythmus gehalten (Licht an, ab 8.00 Uhr), die Raumtemperatur beträgt 22 -24 °C.

Die Tiere werden direkt vor Versuchsbeginn gewogen. Für die intravenöse Administration wurden die erfindungsgemäßen Substanzen in einem Gemisch von Transcutol (GATTEFOSSE GmbH) verdünnt mit 20% Cremophor (BASF) und Wasser im Verhältniss von 3/7 gelöst. Natriumnitroprussid wurde in 0,9% NaCl gelöst. Die Substanzen wurden in den in der Tabelle angegeben Dosierungen in einem Volumen von 0,5 ml /kg die Ohrvene injeziert. Für die orale Gabe wurden die Testsubstanzen in einer Mischung aus Glycerin: Wasser: Polyethylenglykol 6:10:9,69 gelöst und in den in der Tabelle angegebenen Dosierungen in einem Volumen von 1 ml/kg mit der Schlundsonde appliziert.

DieWirkung von Guanylatcyclasestimulatoren wird durch NO-Donatoren verstärkt. Dies wurde mit der zusätzlichen Gabe von Natriumnitroprussid demonstriert.

Das Natriumnitroprussid wurde in einer Dosierung von 0,2 mg/kg gleichzeitig mit der erfindungsgemäßen Substanz in die Ohrvene injeziert. Wurde die erfindungsgemäße Substanz oral gegeben so wurde diesen Tieren das Natriumnitroprussid 30 min. nach der oralen Gabe in die Ohrvene injiziert. Entsprechende Kontrollen mit dem Lösungsmittel und mit Natriumnitroprussid alleine wurden durchgeführt.

Unter Ruhebedingungen ist der Kaninchenpenis in der Schamregion nicht sichtbar und von der Penishaut vollständig bedeckt. Die Erektion wird gewertet, in dem man die Länge des hervortretenden Penis mit einer Schiebelehre misst. Die Messung wird 5, 10, 15, 30, 45, 60min. 120 und 180 min. nach Verabreichung der Substanz durchgefürt. Die Wirkung wird als Produkt der Länge des nicht von Fell bedeckten Penis in [mm]und der Zeit die die Erektion anhält in [min.] berechnet.

Die intravenöse Injektion von Natriumnitroprussid bewirkt eine ca 10 min.anhaltende Erektion (110 [mm x min.]).

**Tabelle 5:**

| **Erektionsfördernde Wirkung von Guanylatcyclase-Stimulatoren (Einheit: [mm] x [min])** | | | | | | |
|---|---|---|---|---|---|---|
| **Substanz** | **Dosis i.v.** | **+SNP i.v.** | **-SNP i.v.** | **Dosis po** | **+SNP po** | **-SNP po** |
| Bsp. 1 | 0,3mg/kg | 140 | 0 | 1mg/kg | 184 | ng |
| | 1mg/kg | 369 | 184 | 3mg/kg | 233 | ng |
| | | | | 10mg/kg | 375 | ng |
| | | | | 30mg/kg | 804 | 418 |
| Bsp.25 | 0,3mg/kg | 85 | 0 | 3mg/kg | 168 | ng |
| | 1mg/kg | 245 | 0 | 10mg/kg | 506 | ng |

### Erläuterungen:

Die Substanzen wurden wie in den angegebenen Dosierungen verabreicht. Das Natriumnitroprussid wurde jeweils i.v.mit 0,2mg/kg gegeben. Wurde die erfindungsgemässe Substanz i.v. gegeben, so wurde das Natriumnitroprussid gleichzeitig verabreicht, bei oraler Gabe der erfindungsgemässen Substanz wurde es 30 min. später gegeben. Alleine bewirkt Natriumnitroprussid einen Effekt von 100 [mm]x[min].

Zur vorliegenden Erfindung gehören pharmazeutische Zubereitungen, die neben nichttoxischen, inerten pharmazeutisch geeigneten Trägerstoffen die erfindungsgemäßen Verbindungen der allgemeinen Formel (I) enthält sowie Verfahren zur Herstellung dieser Zubereitungen.

Die Wirkstoffe können gegebenenfalls in einem oder mehreren der oben angegebenen Trägerstoffe auch in mikroverkapselter Form vorliegen.

Die therapeutisch wirksamen Verbindungen der allgemeinen Formel (I) sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen der allgemeinen Formel (I) auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiele

### Abkürzungen:

- RT:: Raumtemperatur
- EE:: Essigsäureethylester
- BABA:: n-Butylacetat/n-Butanol/Eisessig/Phosphatpuffer pH 6 (50:9:25:15; org. Phase)

### Laufmittel für die Dünnschichtchromatographie:

- T1 E1:: Toluol - Essigsäureethylester (1:1)
- T1 EtOH1:: Toluol - Methanol (1:1)
- C1 E1:: Cyclohexan - Essigsäureethylester (1:1)
- C1 E2:: Cyclohexan - Essigsäureethylester (1:2)

### Ausgangsverbindungen

### Beispiel I: Cyclopropyl-3-oxopropionsäurenitril

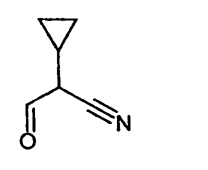

65.4 g (0.583 mol) Kaliumtertbutylat werden in 400 mL THF unter Argon gelöst. Bei Raumtemperatur wird eine Lösung von 21.5 g (0.265 mol) Cyclopropylacetonitril und 43.2 g (0.58 mol) Ethylformiat in 100 mL THF hinzugegeben. Die Reaktion wird 3 Stunden bei RT gerührt. Das THF wird abrotiert und der Rückstand zwischen 200 mL Eiswasser und 200 mL Essigsäureethylester verteilt. Die organische Phase wird verworfen, während die wäßrige Phase mit Salzsäure auf pH=4 gebracht und zweimal mit Essigsäureethylester extrahiert wird. Die organischen Extrakte werden mit Magnesiumsulfat getrocknet und im Vakuum eingedampft. Das Produkt wird roh im Kühlschrank gelagert und weiter umgesetzt.

### Beispiel II: 2-Cyclopropyl-3-dimethylaminoacrylonitril

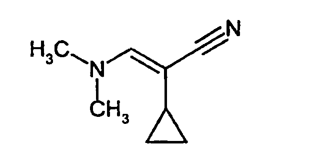

25 g (292.8 mmol) Cyclopropylacetonitril (durch das in US-3,454,575 beschriebene Verfahren erhältlich) werden mit 25.5 g (30.2 ml, 146.4 mmol) Bis(dimethylamino)-tert-butyloxymethan am Steigrohr 46 Stunden bei 100 °C gerührt. Man verdampft leichtflüchtige Komponenten im Vakuum und destilliert anschließend bei 0.1 Torr und 60 - 65°C.
Ausbeute 16.18 g (81 % d.Th.)

Auf analoge Weise zu Beispiel I und II können die entsprechenden Cyclobutyl-, Cyclopentyl-, Cyclohexyl- und 1-Cyclopenten-1-yl-Derivate hergestellt werden.

### Beispiel III: 5-Amino-1-(2-fluorbenzyl)1H-pyrazol-3-carbonsäureethylester

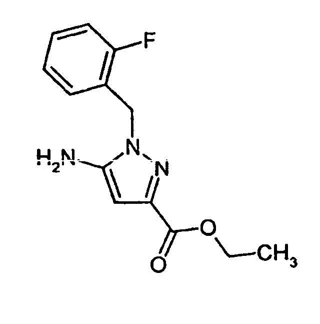

100 g (0.613 mol) Natriumsalz des Cyanobrenztraubensäureethylester (Darstellung analog Borsche und Manteuffel, Liebigs Ann. 1934, *512*, 97) werden unter gutem Rühren unter Argon in 2.5 l Dioxan bei Raumtemperatur mit 111.75 g (75 ml, 0.98 mol) Trifluoressigsäure versetzt und 10 min gerührt, wobei ein großer Teil des Eduktes in Lösung geht. Dann gibt man 85.93 g (0.613 mol) 2-Fluorbenzylhydrazin hinzu und kocht über Nacht. Nach Abkühlen werden die ausgefallenen Kristalle des Natriumtrifluoracetats abgesaugt, mit Dioxan gewaschen und die Lösung roh weiter umgesetzt.

### Beispiel IV: 1-(2-Fluorbenzyl)- 1H-pyrazolo[3,4-b]pyridin-3-carbonsäureethylester

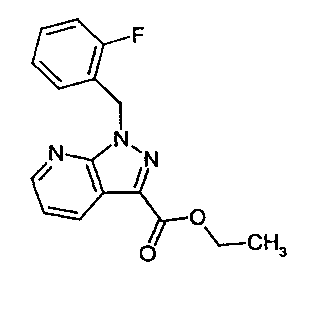

Die aus Beispiel III erhaltene Lösung wird mit 61.25 ml (60.77 g, 0.613 mol) Dimethylaminoacrolein und 56.28 ml (83.88 g, 0.736 mol) Trifluoressigsäure versetzt und unter Argon 3 Tage lang gekocht. Anschließend wird das Lösungsmittel im Vakuum verdampft, der Rückstand in 2 l Wasser gegeben und dreimal mit je 1 l Essigester extrahiert. Die vereinigten organischen Phasen werden mit Magnesiumsulfat getrocknet und einrotiert. Man chromatographiert auf 2.5 kg Kieselgel und eluiert mit einem Toluol / Toluol-Essigester=4:1 -Gradienten. Ausbeute: 91.6 g (49.9 % d.Th. über zwei Stufen).
Smp. 85°C
R_{f}(SiO₂, T1E1): 0.83

### Beispiel V: 1-(2-Fluorbenzyl)-1H-pyrazolo[3,4-b]pyridin-3-carboxamid

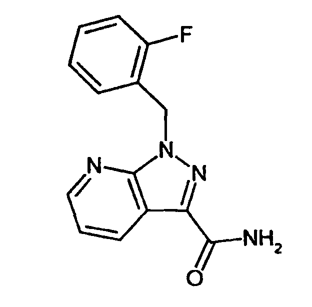

10.18 g (34 mmol) des in Beispiel IV erhaltenen Esters werden in 150 ml mit Ammoniak bei 0 - 10°C gesättigtem Methanol vorgelegt. Man rührt zwei Tage bei Raumtemperatur und engt anschließend im Vakuum ein.
R_{f} (SiO₂, T1E1): 0.33

### Beispiel VI: 3-Cyano-1-(2-fluorbenzyl)-1H-pyrazolo[3,4-b]pyridin

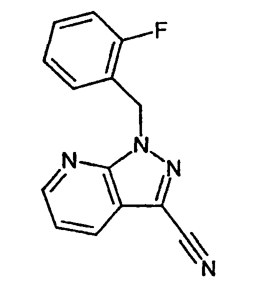

36.1 g (133 mmol) 1-(2-Fluorbenzyl)-1*H*-pyrazolo[3,4-*b*]pyridin-3-carboxamid aus Beispiel V werden in 330 ml THF gelöst und mit 27 g (341 mmol) Pyridin versetzt. Anschließend gibt man innerhalb von 10 min 47.76 ml (71.66 g, 341 mmol) Trifluoressigsäureanhydrid hinzu, wobei die Temperatur bis auf 40 °C ansteigt. Man rührt über Nacht bei Raumtemperatur. Anschließend wird der Ansatz in 1l Wasser gegeben und dreimal mit je 0.5 l Essigester extrahiert. Die organische Phase wird mit gesättigter Natriumhydrogencarbonatlösung und mit 1 N HCl gewaschen, mit MgSO₄ getrocknet und einrotiert.
Ausbeute: 33.7 g (100% d.Th.)
Smp: 81°C
R_{f} (SiO₂, T1E1): 0.74

### Beispiel VII: 1-(2-Fluorbenzyl)-1H-pyrazolo[3,4-b]pyridin-3-carboximidsäure methylester

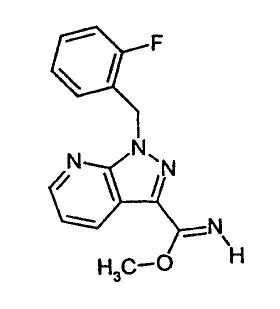

Man löst 30.37 g (562 mmol) Natriummethylat in 1.5 1 Methanol und gibt 36.45 g (144.5 mmol) 3-Cyano-1-(2-fluorbenzyl)-1*H*-pyrazolo[3,4-*b*]pyridin (aus Beispiel VII) hinzu. Man rührt 2 Stunden bei Raumtemperatur und setzt die erhaltene Lösung direkt für die nächste Stufe ein.

### Beispiel VIII: 1-(2-Fluorbenzyl)-1H-pyrazolo[3,4-b]pyridin-3-carboxamidin

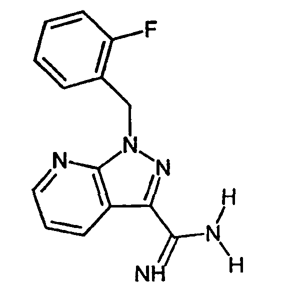

Die aus Beispiel VII erhaltene Lösung von (2-Fluorbenzyl)-1H-pyrazolo[3,4-b]pyridin-3-carboximidsäuremethylester in Methanol wird mit 33.76 g (32.19 ml, 562 mmol) Eisessig und 9.28 g (173 mmol) Ammoniumchlorid versetzt und über Nacht unter Rückfluß gerührt. Man verdampft das Lösungsmittel im Vakuum, verreibt den Rückstand gut mit Aceton und saugt den ausgefallenen Feststoff ab. Man gibt in 2 l Wasser, versetzt unter Rühren mit 31.8 g Natriumcarbonat und extrahiert dreimal mit insgesamt 1 l Essigester, trocknet die organische Phase mit Magnesiumsulfat und dampft im Vakuum ein.
Ausbeute 27.5 g (76.4 % d.Th. über zwei Stufen)
Smp.: 86 °C
R_{f} (SiO₂, T1EtOH1): 0.08

### Beispiel IX: 1-(2-Fluorbenzyl)-3-cyanindazol

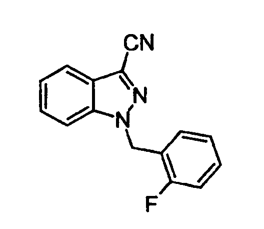

12.0 g (83.9 mmol) 3-Cyanindazol [Herstellung vgl. Salkowski, Chem. Ber. 17 (1884), 508] wurden unter Argon in 100 ml abs. THF gelöst und 20.6 g (109 mmol) 2-Fluorbenzylbromid zugegeben. Unter Eiskühlung wurden portionsweise 2.55 g (100 mmol) Natriumhydrid (95proz.) zugefügt. Nach Rühren über Nacht bei Raumtemperatur wurde am Rotationsverdampfer auf ca. ein Viertel des Volumens eingeengt und mit H₂O und Ethylacetat versetzt. Die wäßrige Phase wurde nochmals mit Ethylacetat extrahiert. Trocknen der vereinigten organischen Phasen über MgSO₄ und Abdestillieren des Lösungsmittels am Rotationsverdampfer lieferte das Produkt.

| | |
|---|---|
| Ausb. | 19.5 g (93%) |
| R_{f}-Wert | 0.69 (Kieselgel; Cyclohexan/Ethylacetat 1:1) |

### Beispiel X: 1-(2-Fluorbenzyl)indazol-3-amidiniumchlorid

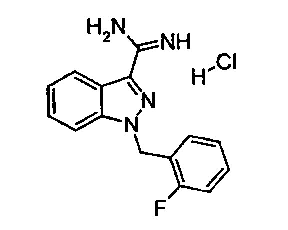

Eine aus 190 mg (8.26 mmol) NaOMe und 30 mi abs. Methanol bereitete Natriummethanolat-Lösung wurde zu einer Lösung aus 20.0g (79.7 mmol) 1-(2-Fluorbenzyl)-3-cyanindazol (aus Beispiel IX) in 200 ml Methanol gegeben und 22 h bei 40°C gerührt. Nach Zugabe von 0.46 ml Essigsäure und 4.30 g NH₄Cl wurde weitere 24 h bei 40°C gerührt und die Mischung anschließend am Rotationsverdampfer zur Trockne eingeengt. Aufnehmen des Rückstands in Aceton und Absaugen des verbleibenden Niederschlags lieferte nach Trocknung im Hochvakuum das Produkt in Form eines hellbeigen Pulvers.

| | |
|---|---|
| Ausb. | 20.5 g (84%) |
| Smp. | > 230°C |
| MS-EI | *m*/*z* (%) = 268 (31, M⁺ der freien Base), 251 (15), 109 (100). |

### Beispiel XI: 2-Cyanocyclopropylacetamid

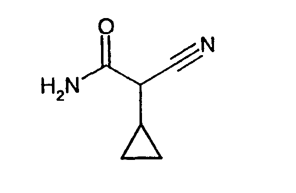

1,5 g (9,8 mmol) 2-Cyanocyclopropylessigsäureethylester (Herstellung nach Li, J. Med. Chem. 1996, 39, 3070) werden in 14 ml methanolischen Ammoniak (7N) gelöst. Nach vier Tagen wird die Lösung einrotiert und der Feststoff mit wenig Methanol nachgewaschen.
Ausbeute: 456 mg (37,5 % d. Th.).
MS (DCI-NH₃): 142 (100 %, M⁺NH₄)

### Beispiel XII: 2-Cyclopropylmalondinitril

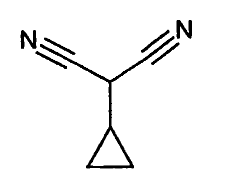

440 mg (3,54 mmol) 2-Cyanocyclopropylacetamid (aus Beispiel XI) werden zweimal mit Toluol abrotiert um das Methanol zu entfernen und die Substanz wird in 15 ml Tetrahydrofuran:Dichlormethan (2:1) gelöst. In drei gleichen Portionen im Abstand von 30 Minuten werden insgesamt 2,53 g (10,63 mmol) des Burgess Reagenzes zugegeben. Nach weiteren dreißig Minuten wird das Reaktionsgemisch direkt an 20 g Kieselgel mit Cyclohexan/Essigsäureethylester (2:1 bis 1:1) chromatographiert.
Ausbeute: 122 mg (32,4 % d. Th.)
R_{f}(SiO₂, C1E1): 0,65

### Beispiel XIII: 1-Cyanmethyl-1-fluormethyl-cyclopropan

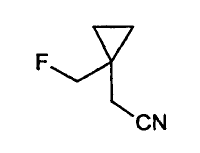

8.85 g (72,2 mmol) 1-Chlormethyl-1-fluormethyl-cyclopropan, 4,60 g (93,8 mmol) Natriumcyanid und 433mg (2,89 mmol) Natriumiodid wurden in 72 ml Triethylenglykol bei 140°C 30 Minuten erhitzt. Die auf Raumtemperatur abgekühlte Mischung wurde mit je 150 ml Dichlormethan und Wasser versetzt, die organische Phase mit gesättigter Natriumchlorid-Lösung gewaschen, über MgSO₄ getrocknet und bei Raumtemperatur am Rotationsverdampfer zur Trockne eingeengt. Das flüssige Produkt wurde ohne weitere Reinigung weiter umgesetzt.
Ausbeute: 5,44 g (67 %)

### Beispiel XIV: 2-[(1-Fluormethyl)cycloprop-1-yl]-2-formylacetonitril

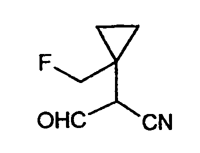

9,20 g (82,1 mmol) Kalium-tert.-butanolat wurde unter Argon in 80 ml wasserfreiem Tetrahydrofuran (THF) vorgelegt und die Mischung mit einer Lösung aus 4,21 g (37,3 mmol) 1-Cyanmethyl-1-fluormethyl-cyclopropan (Bsp. XIII) und 9,24 g (125 mmol) Ethylformiat in 20 ml wasserfreiem THF versetzt. Nach 3-stündigem Rühren bei Raumtemperatur wurde am Rotationsverdampfer zur Trockne eingeengt und der Rückstand mit je 150 ml Eiswasser und Ethylacetat versetzt. Die wäßrige Phase wurde abgetrennt und mit verdünnter Salzsäure auf pH 4 eingestellt. Durch zweifache Extraktion mit je 100 ml Ethylacetat, Trocknen der Organischen Phase über MgSO₄ und Abziehen des Lösungsmittels am Rotationsverdampfer konnte das Produkt isoliert werden. Es wurde ohne weitere Reinigung in der nächsten Reaktion eingesetzt.
Ausbeute: 4,03 g (77 %)

### Herstellungsbeispiele

### Beispiel 1: 3-(4-Amino-5-cyclopropylpyrimidin-2-yl)-1-(2-fluorbenzyl)-1H-pyrazolo[3,4-b]pyridin

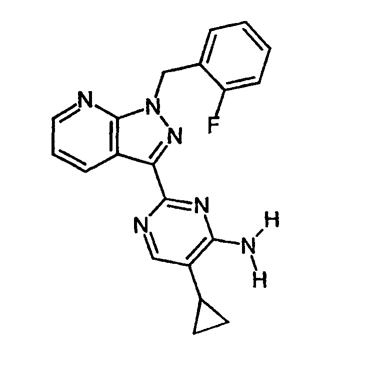

### Vorschrift A

Man zerreibt 2 g (7.4 mmol) des Amidins aus Beispiel VIII, bis es eine puderförmige Konsistenz hat. Das klumpenfreie Edukt wird mit 4 g (29.4 mmol) 2-Cyclopropyl-3-dimethylaminoacrylonitril aus Beispiel II versetzt und mit dem Spatel und Ultraschallbad innigst vermischt, bis eine homogene Milch entstanden ist. Man legt ein Wasserstrahlvakuum an, wobei die Mischung aufschäumt. Anschließend wird der Ansatz unter Umschwenken in ein 106 °C heißes Ölbad getaucht, wobei die Lösung klar wird. Nach 2 Stunden beginnt der Inhalt des Kölbchens sich zu verfestigen. Man läßt am Vakuum über Nacht bei 106 °C. Der entstandene Feststoff wird mit Toluol verrührt, abgesaugt und mit Ether gewaschen. Der Rückstand wird in 50 ml kochendem Acetonitril aufgenommen und abgesaugt. Der hier anfallende Rückstand wird in 25 ml kochendem Dimethylformamid aufgenommen und wieder abgesaugt. Beide Filtrate werden vereinigt und einrotiert.
Ausbeute: 0.85 g (31.2 % d.Th.)
Smp.: 210 °C
MS (ESI-POS): 361 (100%, M+H)
¹H-NMR (300 MHz, d⁶-DMSO): 0.61 (m, 2H, 2-Cyclopropyl), 0.9 (m, 2H, 2-Cyclopropyl), 1.65 (m, 1H, 1-Cyclopropyl), 5.7 (s, 2H, Benzyl-CH₂), 6.98 (breites s, 2H, NH₂), 7.1-7.3 (peak cluster, 3H, aromatische Benzylische H3,5,6), 7.3-7.4 (peak cluster, 2H, H5, Benzylische H4), 8.0 (1H, Pyrimidinyl-H6), 8.6 (d, 1H, H6), 8.95 (d, 1H, H4)

### Vorschrift B

20.0 g (74.3 mmol) des Amidins aus Beispiel VIII und 28.4 g (260.0 mmol) des rohen 2-Cyclopropyl-3-oxopropionsäurenitril aus Beispiel I in 280 mL Toluol werden zusammengeben, im Ultraschallbad vermischt und die Mischung über Nacht zum Rückfluß erhitzt. Die Lösung wird bis zur Hälfte einrotiert, abgekühlt, abgesaugt und mit Essigsäureethylester gewaschen. Das Filtrat wird konzentriert und an Kieselgel 60 (Korngröße 0,040-0,063mm) mit Cyclohexan/Essigester 1:1 als Eluenten chromatographiert. Der Rückstand wird in 50 mL kochendem Toluol aufgenommen und abgesaugt. Beide Lösungen werden vereinigt, einrotiert und mit Toluol rekristallisiert.
Ausbeute: 8.38 g (31.3 % d. Th.).
R_{f} (SiO₂, C1E2): 0.23
Smp:209°C

### Vorschrift C (Methode ohne Lösungsmittel)

103.7 mg (0.38 mmol) des Amidins aus Beispiel VIII und 168.1 mg (1.54 mmol) des rohen 2-Cyclopropyl-3-oxopropionsäurenitril aus Beispiel I in 0.5 mL Toluol werden zusammengeben und im Ultraschallbad vermischt. Das Toluol wird im Vakuum verdampft und die Mischung ohne Lösungsmittel im offenen Gefäß auf 100-105°C erhitzen. Die Mischung wird nach eine Stunde abgekühlt, mit Dichlormethan gelöst, mit 1 g Kieselgel versetzt und einrotiert.

Die Substanz wird zur Reinigung an Kieselgel 60 (Korngröße 0,040-0,063mm) mit Cyclohexan/Essigester 1:1 als Eluenten chromatographiert.
Ausbeute: 50.0 mg (36.0 % d. Th.).
R_{f} (SiO₂, C1E2): 0.23

In Analogie zur den oben aufgeführten Vorschriften erfolgt die Herstellung der folgenden Verbindungen (Bsp. 2 bis 5):

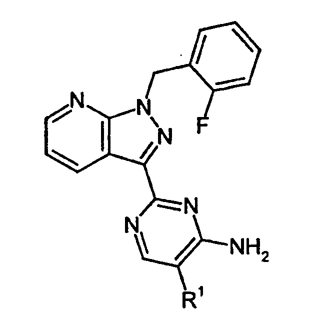

### Beispiel 2: 3-(4-Amino-5-cyclobutylpyrimidin-2-yl)-1-(2-fluorbenzyl)-1H-pyrazolo [3,4-b]pyridin

R¹ = Cyclobutyl
Smp. 214 °C

### Beispiel 3: 3-(4-Amino-5-cyclopentylpyrimidin-2-yl)-1-(2-fluorbenzyl)-1H-pyrazolo [3,4-b]pyridin

R¹ = Cyclopentyl
Smp. 208 °C

### Beispiel 4: 3-(4-Amino-5-cyclohexylpyrimidin-2-yl)-1-(2-fluorbenzyl)-1H-pyrazolo [3,4-b]pyridin

R¹ = Cyclohexyl
Smp. 213°C

### Beispiel 5: 3-(4-Amino-5-(1-cyclopenten-1-yl)-pyrimidin-2-yl)-1-(2-fluorbenzyl)-1H-pyrazolo[3,4-b]pyridin

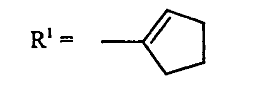

Smp. 228°C.

### Beispiel 6: 3-(4-Amino-5-cyclopropylpyrimidin-2-yl)-1-(2-fluorbenzyl)-1H-pyrazolo[3,4-b]-pyridin, Hydrochlorid

Man löst 0.3 g (0.83 mmol) 3-(4-Amino-5-cyclopropylpyrimidin-2-yl)-1-(2-fluorbenzyl)-1H-pyrazolo[3,4-b]pyridin aus Beispiel 1 in 50 ml heißem Acetonitril und gibt 0.9 ml 1 N HCl (0.9 mmol) hinzu. Anschließend werden die ausgefallenen Kristalle abgesaugt.
Ausb. 0.24 g (72.7 % d.Th.)
Smp. 279 °C.

### Beispiel 7: 3-(4-Amino-5-cyclopropylpyrimidin-2-yl)-1-(2-fluorbenzyl)-1H-pyrazolo[3,4-b]-pyridin, Tosylat

Man löst 0.3 g 3-(4-Amino-5-cyclopropylpyrimidin-2-yl)-1-(2-fluorbenzyl)-1H-pyrazolo[3,4-b]pyridin aus Beispiel 1 in 50 ml heißen Acetonitril und gibt 160 mg p-Toluolsulfonsäure hinzu. Nach Abkühlen auf Raumtemperatur saugt man die ausgefallenen Kristalle ab.
Ausbeute: 303 mg (68.8 % d.Th.),
Smp. 203 °C

### Beispiel 8: 3-[4-(2-(Benzoyloxymethyl)benzoylamino)-5-cyclopropylpyrimidin-2-yl]-1-(2-fluorbenzyl)-1H-pyrazolo[3,4-b]pyridin

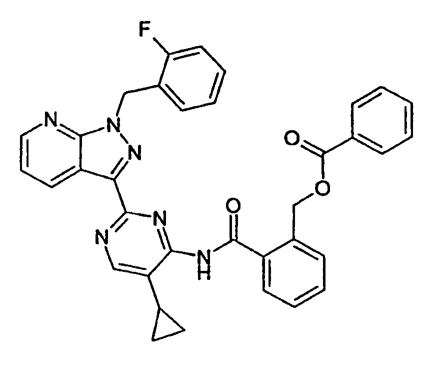

105 mg (2.62 mmol) Natriumhydrid (60%ig ölige Suspension) wird bei Raumtemperatur in 20 mL THF suspendiert. 472 mg (1.31 mmol) des Amins aus Beispiel 1 in 10 mL THF und 432 mg (1.57 mmol) 2-(Benzoyloxymethyl)benzoylchlorid in 5 mL THF werden zugegeben. Die Lösung wird mit Wasser versetzt, mit Essigsäureethylester extrahiert und mit 1M Salzsäure und NaHCO₃ Lösung gewaschen, mit Magnesiumsulfat getrocknet und im Vakuum eingedampft. Die Substanz wird mehrfach mit Cyclohexan digeriert.
Ausbeute: 474 mg (60.4 % d. Th.)
Rf: 0.61 (SiO₂, C1E2)
Smp: 134-136°C

### Beispiel 9: 3-(5-Cyclopropyl-4-(2,2-dimethylpropanoyl)aminopyrimidin-2-yl)-1-(2-fluorbenzyl)-1H-pyrazolo[3,4-b]pyridin

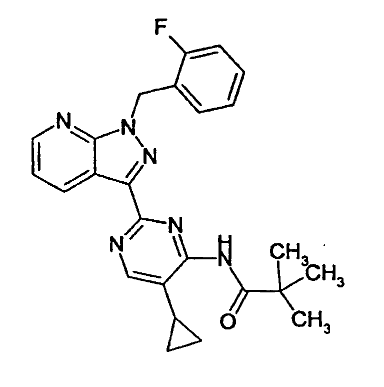

60 mg (0.17 mmol) des Amins aus Beispiel 1 werden in Dichlormethan aufgenommen, 30 mg (0.25 mmol) Pivaloylchlorid, 26 mg (0.33 mmol) Pyridin und eine katalytische Menge Dimethylaminopyridin werden zugeben und die Lösung wird vier Stunden bei Raumtemperatur gerührt. Die Lösung wird mit 1N Salzsäure und gesättiger NaHCO₃ Lösung gewaschen, mit MgSO₄ getrocknet und im Vakuum eingedampft. Das Rohprodukt wird auf Kieselgel mit Cyclohexan/Essigester 1:1 als Eluenten chromatographiert.
Ausbeute: 24.8 mg (33.5% d. Th.)
Rf: 0.59 (SiO₂, EE)

Auf analoge Weise sind erhältlich:

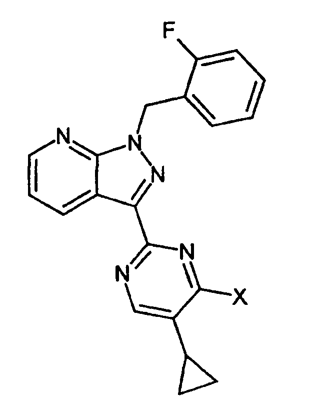

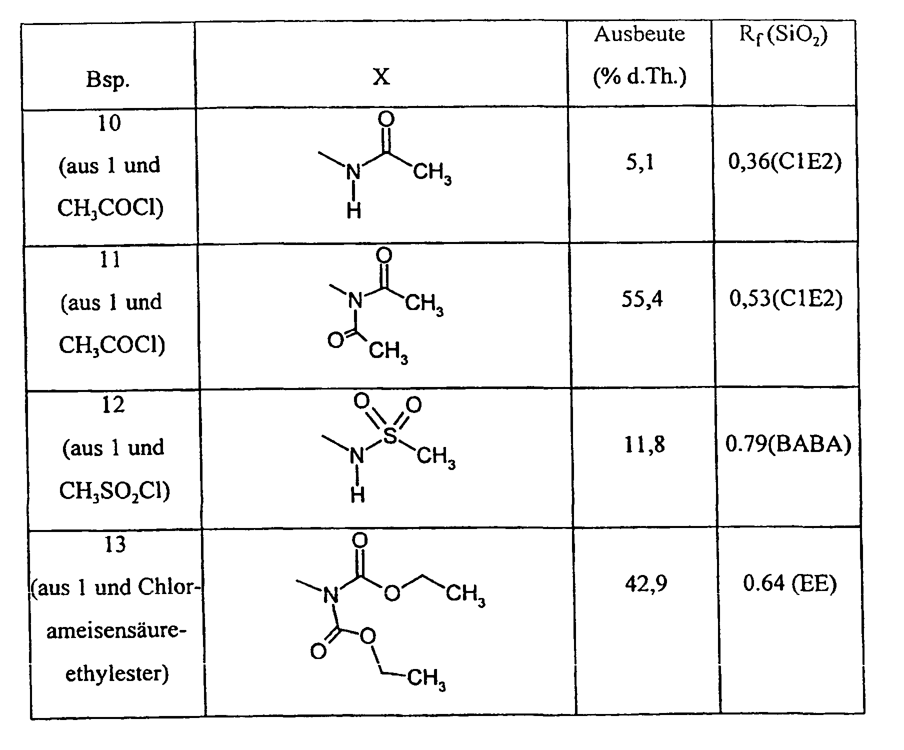

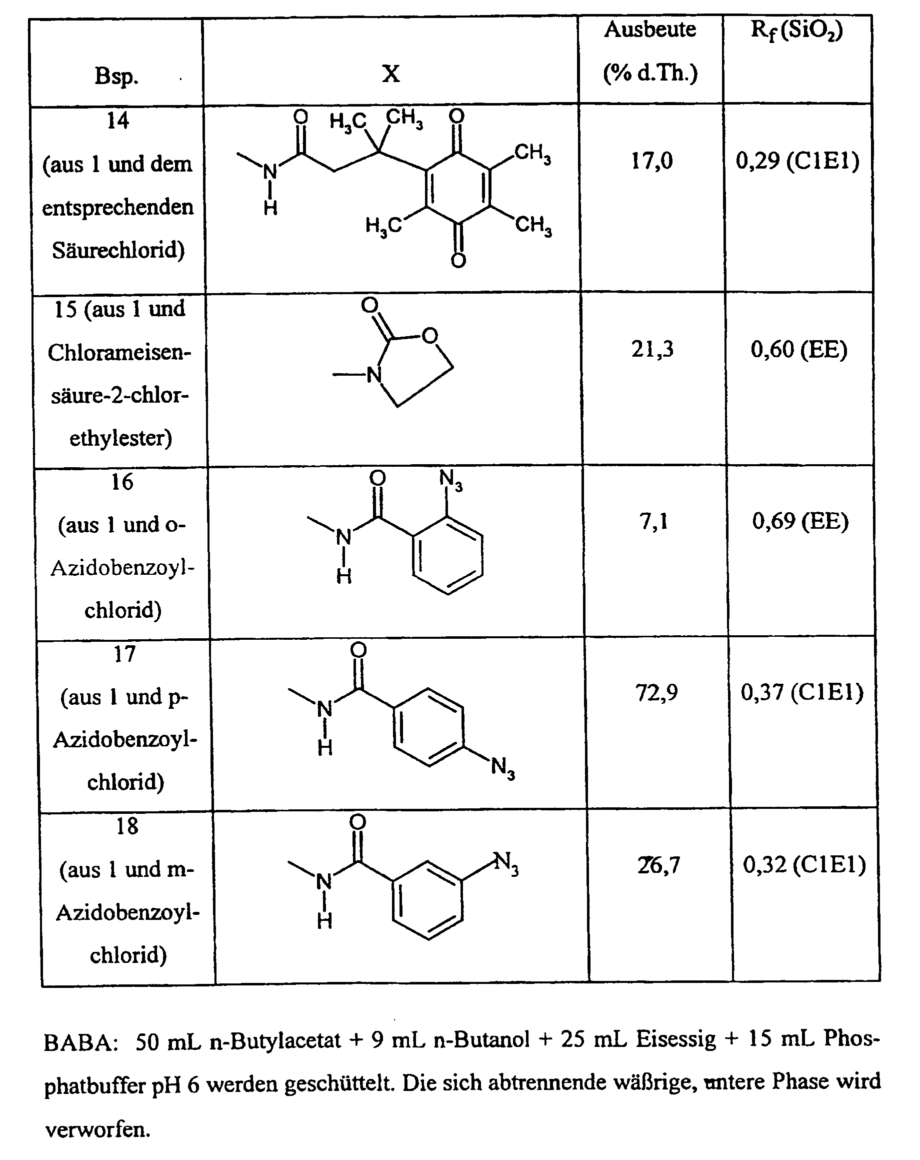

### Beispiel 19: 3-(5-Cyclopropyl-4-(ethoxymethylamino)pyrimidin-2-yl)-1-(2-fluorbenzyl)-1H-pyrazolo[3,4-b]pyridin

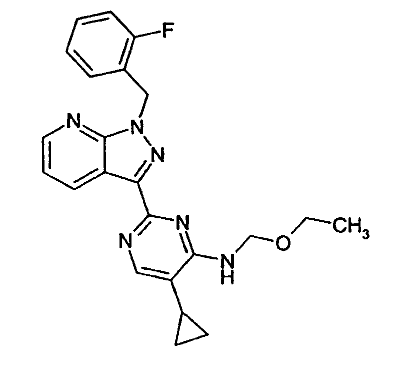

195.5 mg (0.54 mmol) des Amins aus Beispiel 1 werden in 5 mL Ethanol gelöst. 0.9 mL (10.85 mmol) 37%ig Formaldehydlösung werden zugegeben und die Lösung wird 24 Stunden zum Rückfluß erhitzt. Die Lösungsmittel werden im Vakuum eingedampft und das Rohprodukt wird auf Kieselgel mit Cyclohexan/Essigester 2:1 bis Essigester chromatographiert.
Ausbeute: 87.1 mg (38.4 % d. Th.)
Rf: 0.51 (SiO₂, EE)

Analog kann dargestellt werden:

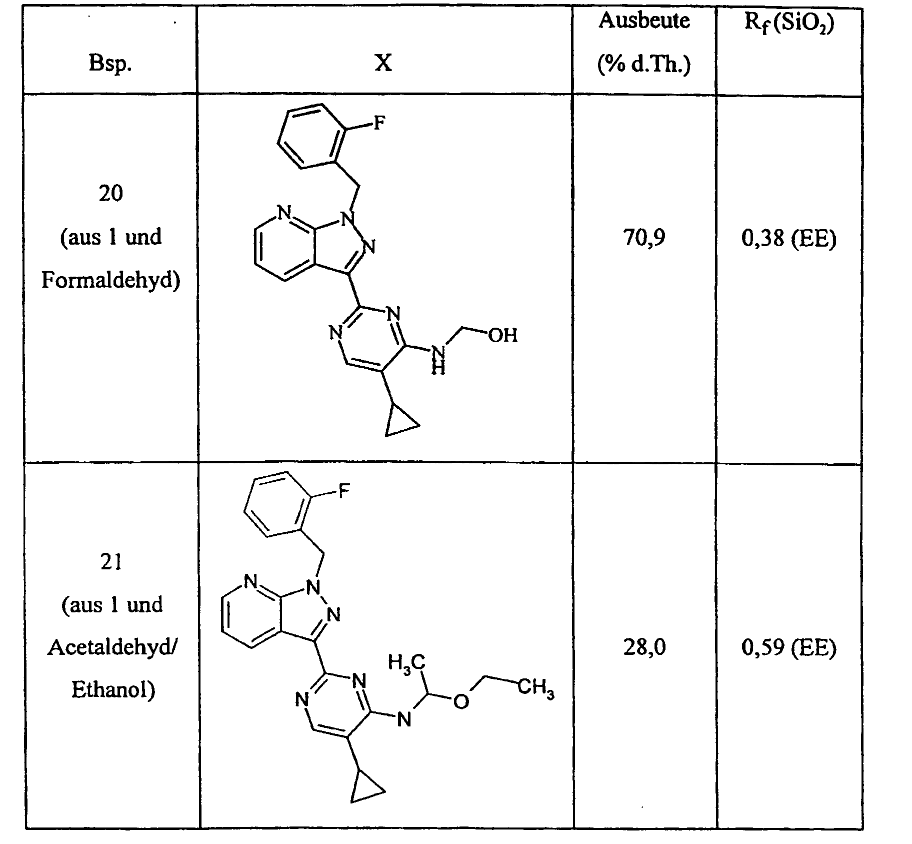

### Beispiel 22: 3-(5-Cyclopropyl-4-(propylaminocarbonylamino)pyrimidin-2-yl)-1-(2-fluorbenzyl)-1H-pyrazolo[3,4-b]pyridin

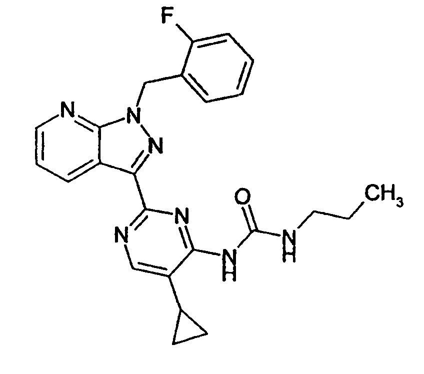

106 mg (0.29 mmol) des Amins aus Beispiel 1 werden unter Argon in Dichlormethan suspendiert und bei 0°C mit 137.6 mg (1.62 mmol) Propylisocyanat versetzt. Die Mischung wird zum Rückfluß 38 Stunden erhitzt. Die Substanz wird auf Kieselgel mit Cyclohexan/Essigester 1:1 bis Essigester als Eluenten chromatographiert.
Ausbeute: 52 mg (39.6% d. Th.)
Rf: 0.58 (SiO₂, EE)

Analog können dargestellt werden:

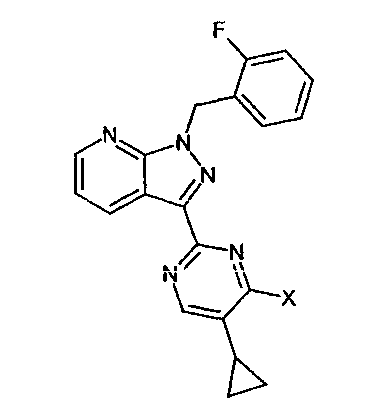

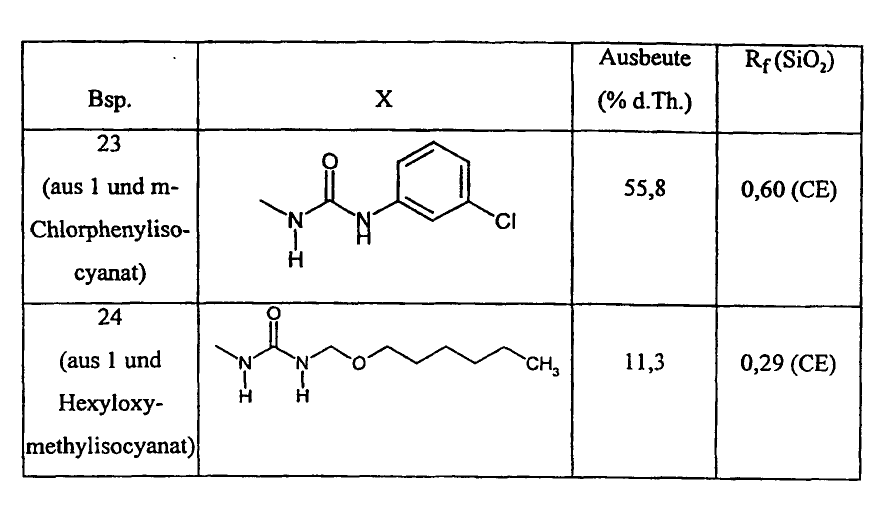

### Beispiel 25 : 3-(4-Amino-5-cyclopropyl-2-pyrimidyl)-1-(2-fluorbenzyl)indazol

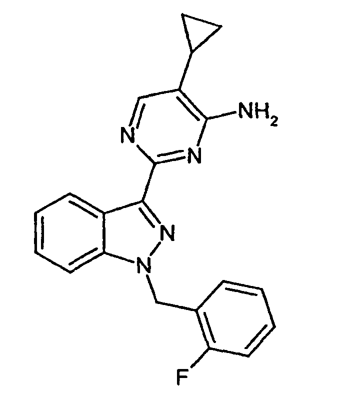

Unter Argon wurden 1.08 g (6.00 mmol) Natriummethanolat-Lösung (30proz. in Methanol) mit 15 ml abs. Methanol und 1.83 g (6.00 mmol) 1-(2-Fluorbenzyl)indazol-3-amidiniumchlorid aus Beispiel X versetzt. Nach 5-minütigem Rühren bei Raumtemperatur wurden 816 mg (6.00 mmol) 2-Cyclopropyl-3-dimethylaminoacrylnitril aus Beispiel II zugegeben und über Nacht unter Rückfluß erhitzt. Nach Abkühlen auf Raumtemperatur wurde der Niederschlag abgesaugt und in Pentan verrührt. Erneutes Absaugen des Niederschlags und Trocknen im Hochvakuum lieferte das Produkt in Form eines nahezu weißen Feststoffs.

| | |
|---|---|
| Ausb. | 700 mg (32%) |
| Smp. | 218°C |

¹H-NMR: (400 MHz, D₆-DMSO), δ = 0.61 (m, 2H, Cyclo-Pr-CH₂), 0.91 (m, 2H, Cyclo-Pr-CH₂), 1.67 (m, 1H, Cyclo-Pr-CH), 5.80 (s, 2H, CH₂), 6.99 (br. s, 2H, NH₂), 7.04 - 7.13 (m, 2H, Ar-H), 7.20 - 7.27 (m, 2H, Ar-H), 7.31 - 7.38 (m, 1H, Ar-H), 7.43 (t, 1H, Ar-H), 7.73 (d, 1H, Ar-H), 7.99 (s, 1H, Ar-H), 8.63 (d, 1H, Ar-H).

### Beispiel 26: 3-(4,6-Diamino-5-cyclopropylpyrimidin-2-yl)-1-(2-fluorbenzyl)-1H-pyrazolo[3,4-b]pyridin

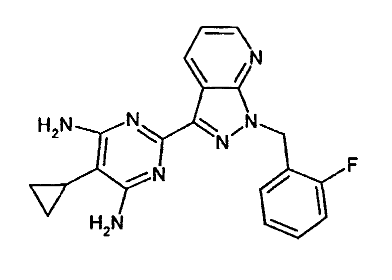

155 mg (0,57 mmol) des Amidins aus Beispiel VIII und 122 mg (1,15 mmol) 2-Cyclopropylmalondinitril aus Bsp. XII werden in Dichlormethan zusammengegeben und im offenen Gefäß auf 105°C erhitzt. Nach einer Stunde wird die Reaktionsmischung fest. Nach drei Stunden wird die Mischung abgekühlt, mit Toluol digeriert, filtriert und bei Hochvakuum getrocknet.

| | |
|---|---|
| Ausb. | 156 mg (72,3 % d. Th.) |
| R_{f} (SiO₂, BABA) | 0,37 |
| Smp. | 198-200°C |

In Analogie zu Beispiel 26 erhält man die folgenden Verbindungen:

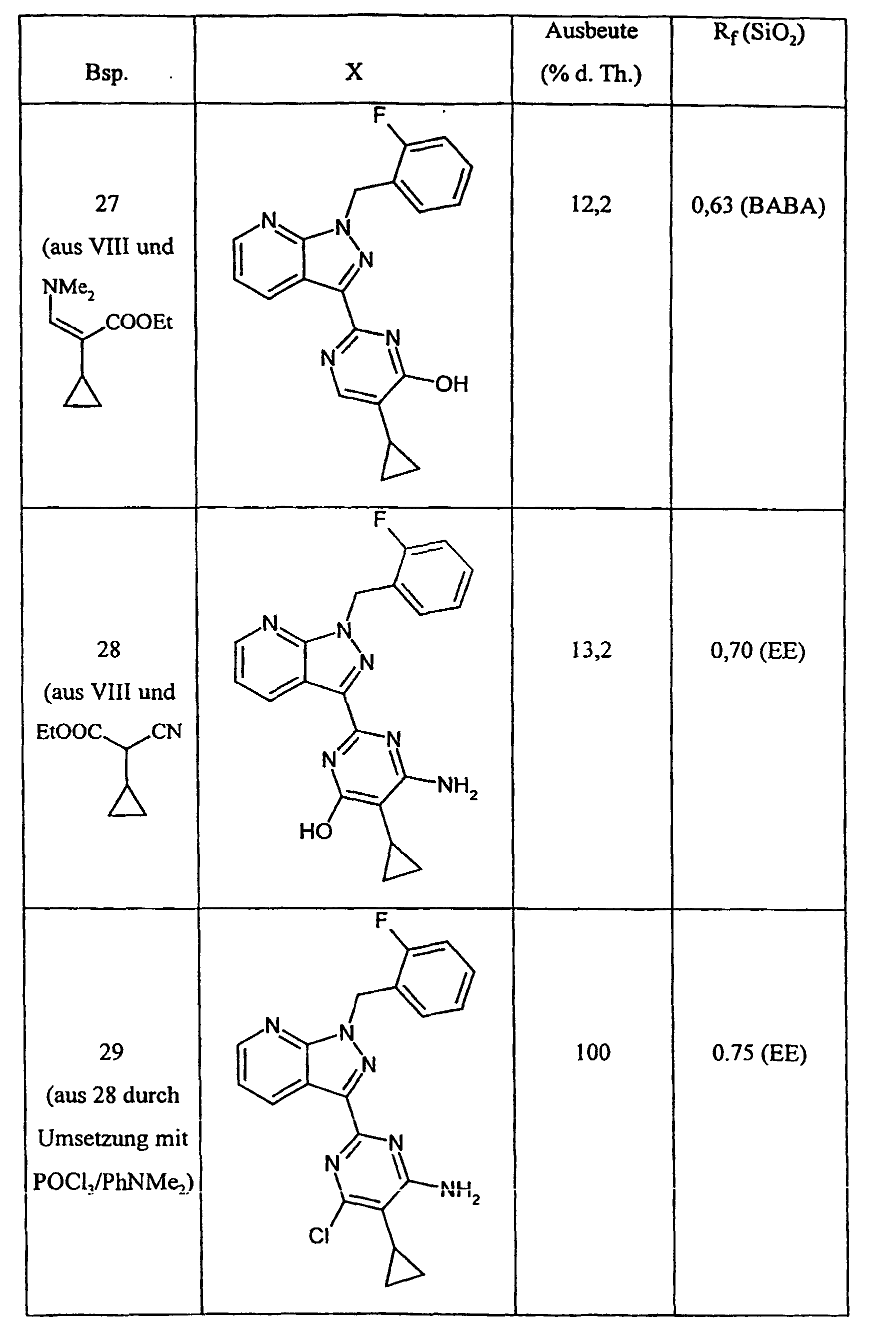

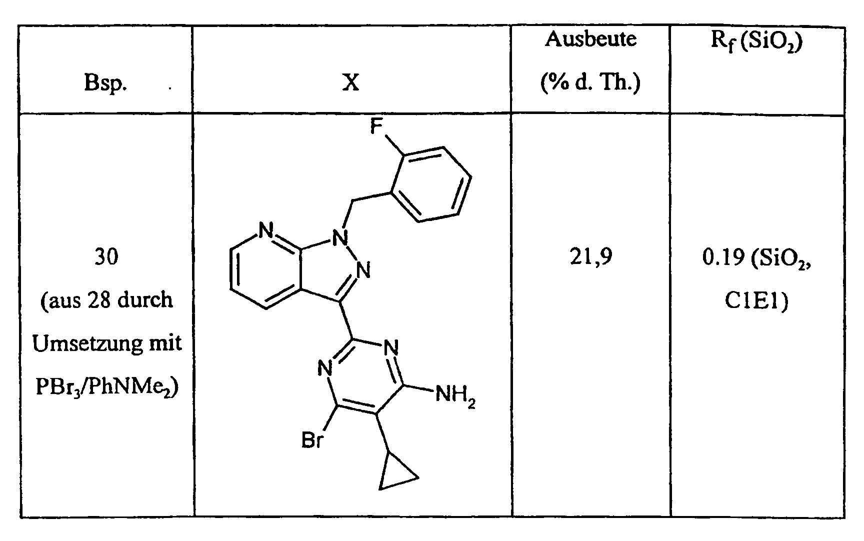

### Beispiel 31: 3-[4-Amino-5-(1-Fluormethyl)cycloprop-1-yl]-pyrimidin-2-yl)-1-(2-fluorbenzyl)-1H-pyrazolo[3,4-b]pyridin

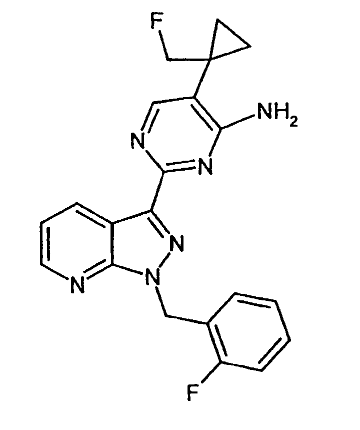

1,00 g (3,70 mmol) des Amidins aus Bsp. VIII und 1,83 g (13,0 mmol) 2-[(1-Fluormethyl)cycloprop-1-yl]-2-formylacetonitril aus Bsp. XIV wurden in 30 ml Toluol suspendiert. Die Mischung wurde 5 Minuten mit Ultraschall beschallt und anschließend unter Rückfluß erhitzt. Nach Einengen am Rotationsverdampfer wurde an Kieselgel chromatographiert (C → C1:E1 → EE). Die polarste Fraktion mußte einer weiteren Säulenchromatographie unterzogen werden, wobei wiederum mit Gradient (C/EE) gearbeitet wurde, aber dem Eluens jeweils 1 % Triethylamin zugesetzt wurde.

| | |
|---|---|
| Ausbeute | 486 mg (34 % d. Th.) |
| Smp. | 220°C |
| R_{f} Wert | 0,19 (C1:E1) |

## Patentansprüche

1. Substituierte Pyrazolderivate der allgemeinen Formel (I), 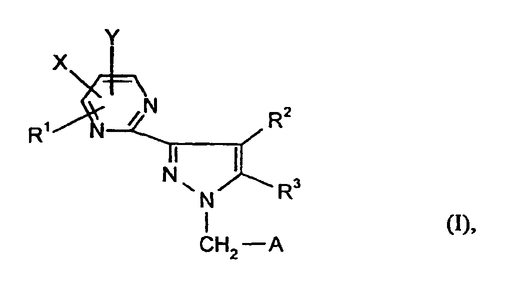 in welcher
R² und R³ unter Einbezug der Doppelbindung einen Pyridylring bilden, so daß sich ein Pyrazolo[3,4-b]pyridin Grundkörper ergibt,
A für 2-Fluorphenyl steht,
R¹, X und Y wie folgt an den Pyrimidinring gebunden sind 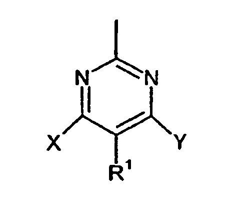 und
R¹ für einen Cyclopropyl-, Cyclobutyl-, Cyclopentenyl-, Cyclopentyl-, Cyclohexyl-, 1-Hydroxycyclopropyl- oder 1-(Fluormethyl)cyclopropylrest steht
X für NH₂ steht,
Y für Wasserstoff oder NH₂ steht,
und ihre physiologisch unbedenklichen Salze.

2. Verbindungen nach Anspruch 1, wobei R¹ für einen Cyclopropylrest steht.

3. Verfahren zur Herstellung der Verbindungen der allgemeinen Formel (I) nach Anspruch 1,
**dadurch gekennzeichnet, daß**,
man in Abhängigkeit der verschiedenen Bedeutungen der oben unter R² und R³, aufgerührten Heterocyclen
[A] Verbindungen der allgemeinen Formel (II) 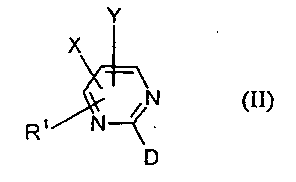 in welcher
R¹, X und Y die oben angegebene Bedeutung haben,
und
D für Reste der Formeln 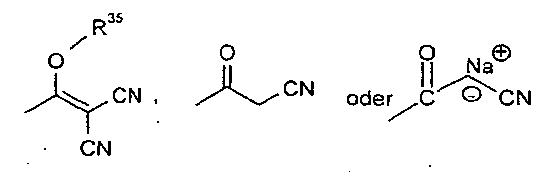 steht,
in welchen
R³⁵ für C₁-C₄-Alkyl steht,
durch Umsetzung mit Verbindungen der allgemeinen Formel (III)
A-CH₂-NH-NH₂ (III)
in
A die oben angegebene Bedeutung hat
in inerten Lösemitteln, gegebenenfalls in Anwesenheit einer Base, in die Verbindungen der allgemeinen Formel (IV) oder (IVa) 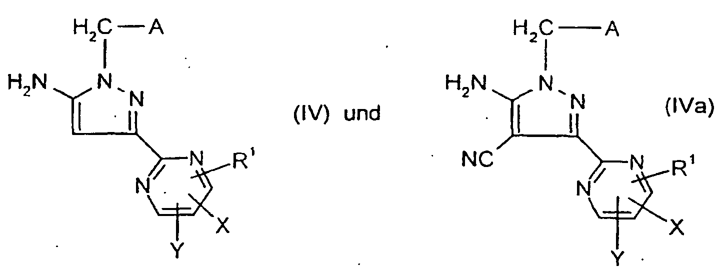
in welcher
A, X, Y und R¹ die oben angegebene Bedeutung haben,
überführt,
und im Fall der Verbindungen der allgemeinen Formel (IVa) anschließend mit Carbonsäuren, Nitrilen, Formamiden oder Guanidiniumsalzen cyclisiert,
und im Fall der Verbindungen der allgemeinen Formel (IV) mit 1,3-Dicarbonyl-Derivaten, deren Salze, Tautomeren, Enolether oder Enaminen, in Anwesenheit von Säuren und gegebenenfalls unter Mikrowellen cyclisiert,
oder
[B] Verbindungen der allgemeinen Formel (VII) 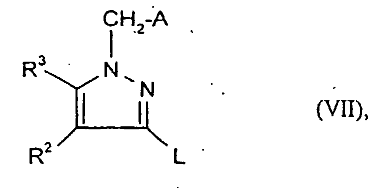 in welcher
A¹, R² und R³ die oben angegebene Bedeutung haben,
und
L für einen Rest der Formel -SnR³⁶R³⁷R³⁸, ZnR³⁹, Iod, Brom oder Triflat steht,
worin
R³⁶, R³⁷ und R³⁸ gleich oder verschieden sind und geradkettiges oder verzweigtes Alkyl mit bis zu 4 Kohlenstoffatomen bedeuten,
und
R³⁹ Halogen bedeutet,
mit Verbindungen der allgemeinen Formel (VIII) 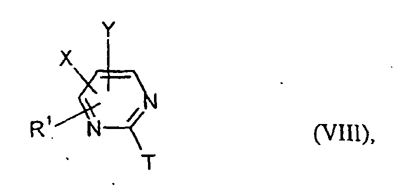 in welcher
X, Y und R¹ die oben angegebene Bedeutung haben
und
im Fall L = SnR³⁶R³⁷R³⁸ oder ZnR³⁹
T für Triflat oder für Halogen, vorzugsweise für Brom steht,
und
im Fall L = Iod, Brom oder Triflat
T für einen Rest der Formel SnR^{36'}R^{37'}R^{38'}, ZnR^{39'} oder BR⁴⁰R⁴¹ steht,
worin
R^{36'}, R^{37'}, R^{38'} und R^{39'} die oben angebene Bedeutung von R³⁶, R³⁷, R³⁸ und R³⁹ haben und mit diesen gleich oder verschieden sind,
R⁴⁰ und R⁴¹ gleich oder verschieden sind und Hydroxy, Aryloxy mit 6 bis 10 Kohlenstoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden,
in einer palladiumkatalysierten Reaktion in inerten Lösemitteln umsetzt, gegebenenfalls in Gegenwart einer Base,
oder
[C] Amidine der allgemeinen Formel (IX) 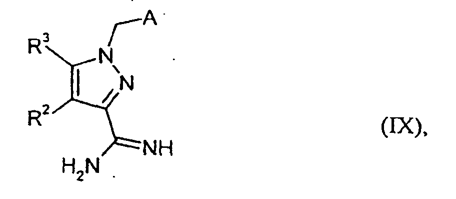 in welcher
A, R² und R³ die oben angegebene Bedeutung haben,
beispielsweise mit Verbindungen der allgemeinen Formel (X), (Xa), (Xb) oder (Xc) 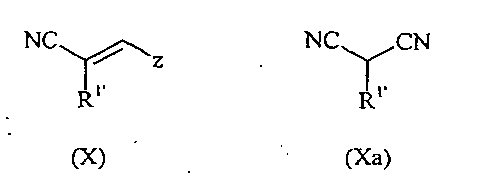 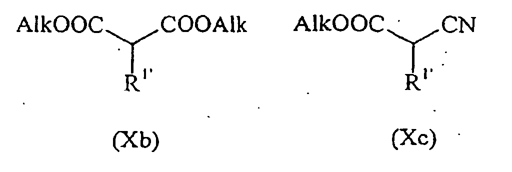 in welchen
R^{1'} für den oben unter R¹ aufgeführten gegebenenfalls substituierten Cycloalkylrest steht;
Alk für geradkettiges oder verzweigtes Alkyl mit bis zu 8 Kohlenstoffatomen, vorzugsweise bis zu vier Kohlenstoffatomen steht;
und
Z für eine NH₂-Gruppe, Monoalkylaminogruppe mit bis zu 7 Kohlenstoffatomen, Dialkylaminogruppe mit bis zu 7 Kohlenstoffatomen, einen über den Stickstoff gebundenen Piperidino- oder Morpholinorest, Hydroxyl, Alkoxy mit bis zu 7 Kohlenstoffatomen, Acyloxy mit bis zu 7 Kohlenstoffatomen oder Aroyloxy mit 6 bis 10 Kohlenstoffatomen steht,
umsetzt,
und gegebenenfalls die unter X, Y, R¹, R², R³ und/oder A aufgeführten Substituenten nach üblichen Methoden, vorzugsweise durch Acylierung und Derivatisierung freier Aminogruppen, Chlorierung, katalytische Hydrierung, Reduktion, Oxidation, Abspaltung von Schutzgruppen und/oder nucleophiler Substitution variiert oder einführt.

4. Amidine der allgemeinen Formel (IX) 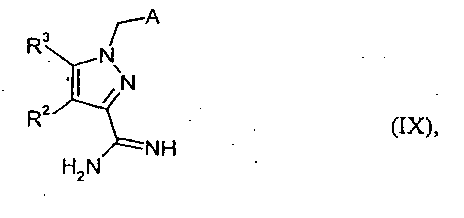 in welcher
R², R³ und A wie in Anspruch 1 definiert sind
und deren isomere Formen und Salze.

5. Arzneimittel enthaltend mindestens eine Verbindung der allgemeinen Formel (I) gemäß Anspruch 1.

6. Verfahren zur Herstellung von Arzneimitteln **dadurch gekennzeichnet, daß** man mindestens eine Verbindung der Formel (I) gemäß Anspruch 1, gegebenenfalls mit üblichen Hilfs- und Zusatzstoffen in eine geeignete Applikationsform überführt.

7. Arzneimittel enthaltend mindestens eine Verbindung der allgemeinen Formel (I) gemäß Anspruch 1 in Kombination mit organischen Nitraten oder NO-Donatoren.

8. Arzneimittel enthaltend mindestens eine Verbindung der allgemeinen Formel (I) gemäß Anspruch 1 in Kombination mit Verbindungen, die den Abbau von cyclischen Guanosinmonophosphat (cGMP) inhibieren.

9. Verwendung von Verbindungen der allgemeinen Formel (I) gemäß Anspruch 1 zur Herstellung von Arzneimitteln.

10. Verwendung von Verbindungen der allgemeinen Formel (I) gemäß Anspruch 1 bei der Herstellung von Arzneimitteln zur Behandlung von Herz-Kreislauf-Erkrankungen.

11. Verwendung von Verbindungen der allgemeinen Formel (I) gemäß Anspruch 1 bei der Herstellung von Arzneimitteln zur Behandlung von Hypertonie.

12. Verwendung von Verbindungen der allgemeinen Formel (I) gemäß Anspruch 1 bei der Herstellung von Arzneimitteln zur Behandlung von thromboembolischen Erkrankungen und Ischämien.

13. Verwendung von Verbindungen der allgemeinen Formel (I) gemäß Anspruch 1 bei der Herstellung von Arzneimitteln zur Behandlung von sexueller Dysfunktion.

14. Verwendung von Verbindungen der allgemeinen Formel (I) gemäß Anspruch 1 bei der Herstellung von Arzneimitteln mit antiinflammatorischen Eigenschaften.

15. Verwendung gemäß einem der Ansprüche 9 bis 14, wobei die Verbindungen der allgemeinen Formel (I) gemäß Anspruch 1 in Kombination mit organischen Nitraten oder NO-Donatoren oder in Kombination mit Verbindungen, die den Abbau von cyclischen Guanosinmonophosphat (cGMP) inhibieren, eingesetzt werden.

## Claims

1. Substituted pyrazole derivatives of the general formula (I) 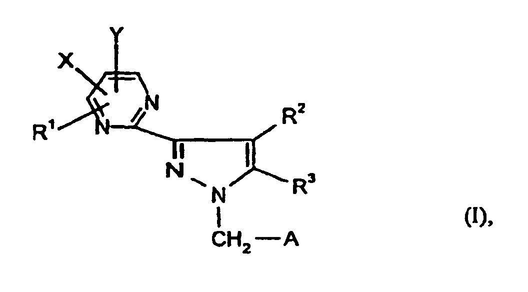 in which
R² and R³, together with the double bond, form a pyridyl ring, resulting in a pyrazolo[3,4-b]pyridine skeleton,
A represents 2-fluorophenyl,
R¹, X and Y are attached to the pyrimidine ring as follows 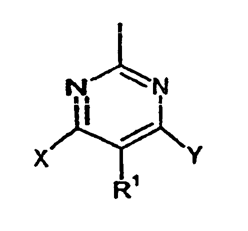
and
R¹ represents a cyclopropyl, cyclobutyl, cyclopentenyl, cyclopentyl, cyclohexyl, 1-hydroxycyclopropyl or 1-(fluoromethyl)cyclopropyl radical,
X represents NH₂,
Y represents hydrogen or NH₂,
and their physiologically acceptable salts.

2. Compounds according to Claim 1 where R¹ represents a cyclopropyl radical.

3. Process for preparing compounds of the general formula (I) according to Claim 1,
**characterized in that**,
depending on the various meanings of the heterocycles listed above under R² and R³,
[A] compounds of the general formula (II) 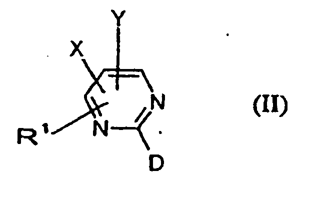 in which
R¹, X and Y are as defined above,
and
D represents radicals of the formulae 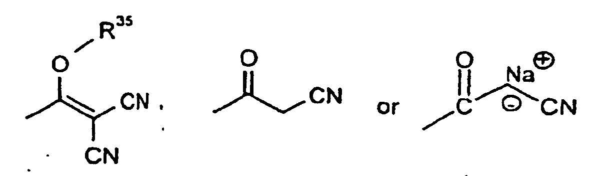 in which
R³⁵ represents C₁-C₄-alkyl,
are converted, by reaction with the compounds of the general formula (III)
A-CH₂-NH-NH₂ (III)
in which
A is as defined above
in inert solvents, if appropriate in the presence of a base, into the compounds of the general formula (IV) or (IVa) 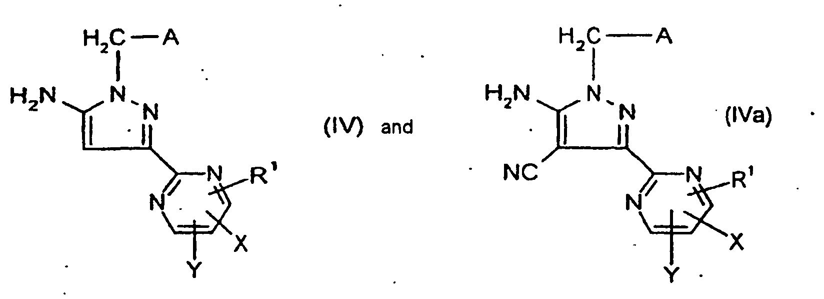
in which
A, X, Y and R¹ are as defined above,
and, in the case of the compounds of the general formula (IVa), are subsequently cyclized with carboxylic acids, nitriles, formamides or guanidinium salts,
and, in the case of the compounds of the general formula (IV), are cyclized with 1,3-dicarbonyl derivatives, their salts, tautomers, enol ethers or enamines, in the presence of acids and, if appropriate, under microwave radiation,
or
[B] compounds of the general formula (VII) 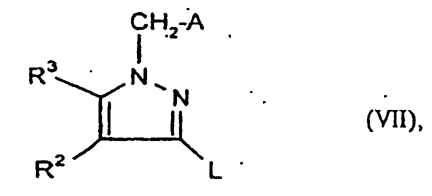 in which
A¹, R² and R³ are as defined above,
and
L represents a radical of the formula -SnR³⁶R³⁷R³⁸, ZnR³⁹, iodine, bromine or triflate,
in which
R³⁶, R³⁷ and R³⁸ are identical or different and represent straight-chain or branched alkyl having up to 4 carbon atoms,
and
R³⁹ represents halogen,
are reacted with compounds of the general formula (VIII) 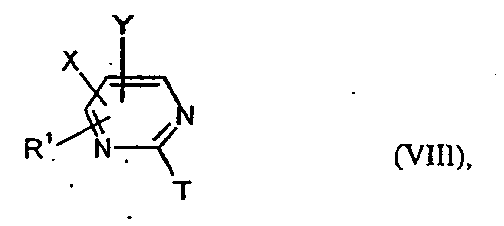 in which
X, Y and R¹ are as defined above,
and,
if L = SnR³⁶R³⁷R³⁸ or ZnR³⁹,
T represents triflate or represents halogen, preferably bromine,
and,
if L = iodine, bromine or triflate,
T represents a radical of the formula SnR^{36,}R^{37,}R^{38,}, ZnR^{39,} or BR⁴⁰R⁴¹,
in which
R^{36'}, R^{37'}, R^{38'} and R^{39'} have the meanings of R³⁶, R³⁷ R³⁸ and R³⁹ given above and are identical to or different from these radicals,
R⁴⁰ and R⁴¹ are identical or different and represent hydroxyl, aryloxy having 6 to 10 carbon atoms or straight-chain or branched alkyl or alkoxy having in each case up to 5 carbon atoms, or together form a 5- or 6-membered carbocyclic ring,
in a palladium-catalysed reaction in inert solvents, if appropriate in the presence of a base,
or
[C] amidines of the general formula (IX) 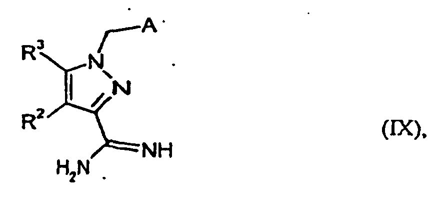 in which
A, R² and R³ are as defined above,
are reacted, for example, with compounds of the general formula (X), (Xa), (Xb) or (Xc) 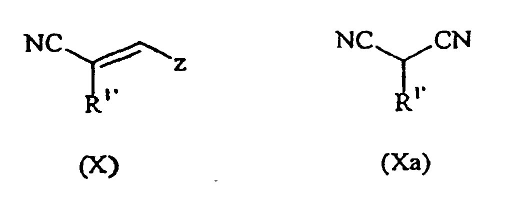 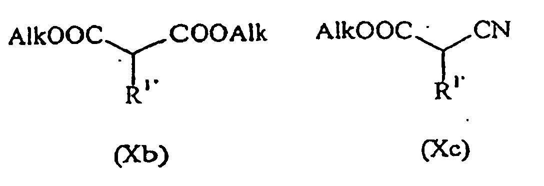 in which
R^{1'} represents the optionally substituted cycloalkyl radical listed above under R¹;
Alk represents straight-chain or branched alkyl having up to 8 carbon atoms, preferably up to 4 carbon atoms;
and
Z represents an NH₂ group, a monoalkylamino group having up to 7 carbon atoms, a dialkylamino group having up to 7 carbon atoms, a piperidino or morpholino radical which is attached via the nitrogen, hydroxyl, alkoxy having up to 7 carbon atoms, acyloxy having up to 7 carbon atoms or aroyloxy having 6 to 10 carbon atoms,
and, if appropriate, the substituents listed under X, Y, R¹, R², R³ and/or A are modified or introduced by customary methods, preferably by acylation and derivatization of free amino groups, chlorination, catalytic hydrogenation, reduction, oxidation, removal of protective groups and/or nucleophilic substitution.

4. Amidines of the general formula (IX) 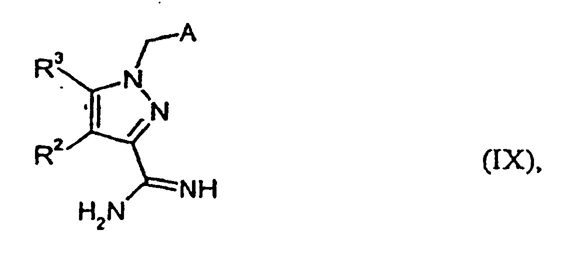 in which
R², R³ and A are as defined in Claim 1,
and their isomeric forms and salts.

5. Medicaments, comprising at least one compound of the general formula (I) according to Claim 1.

6. Process for preparing medicaments, **characterized in that** at least one compound of the formula (I) according to Claim 1, if appropriate with customary auxiliaries and additives, is converted into a suitable administration form.

7. Medicaments, comprising at least one compound of the general formula (I) according to Claim 1 in combination with organic nitrates or NO donors.

8. Medicaments, comprising at least one compound of the general formula (I) according to Claim 1 in combination with compounds which inhibit the degradation of cyclic guanosine monophosphate (cGMP).

9. Use of compounds of the general formula (I) according to Claim 1 for preparing medicaments.

10. Use of compounds of the general formula (I) according to Claim 1 for preparing medicaments for the treatment of cardiovascular diseases.

11. Use of compounds of the general formula (I) according to Claim 1 for preparing medicaments for the treatment of hypertension.

12. Use of compounds of the general formula (I) according to Claim 1 for preparing medicaments for the treatment of thromboembolic disorders and ischaemia.

13. Use of compounds of the general formula (I) according to Claim 1 for preparing medicaments for the treatment of sexual dysfunction.

14. Use of compounds of the general formula (I) according to Claim 1 for preparing medicaments having antiinflammatory properties.

15. Use according to any of Claims 9 to 14, where the compounds of the general formula (I) according to Claim 1 are used in combination with organic nitrates or NO donors or in combination with compounds which inhibit the degradation of cyclic guanosine monophosphate (cGMP).

## Revendications

1. Dérivés substitués de pyrazole de formule générale (I), 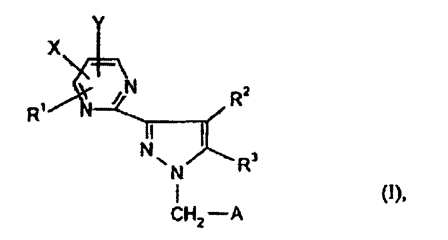 dans laquelle
R² et R³ forment un noyau pyridyle conjointement avec la double liaison, en donnant ainsi comme substance de base une pyrazolo[3,4-b]pyridine,
A est un reste 2-fluorophényle,
R¹, X et Y sont liés comme suit au noyau pyrimidine 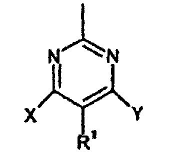 et
R¹ est un reste cyclopropyle, cyclobutyle, cyclopentényle, cyclopentyle, cyclohexyle, 1-hydroxycyclopropyle ou 1-(fluorométhyl)cyclopropyle,
X est un groupe NH₂,
Y représente l'hydrogène ou un groupe NH₂,
et leurs sels acceptables du point de vue physiologique.

2. Composés suivant la revendication 1, dans lesquels R¹ représente un reste cyclopropyle.

3. Procédé de production des composés de formule générale (I) suivant la revendication 1,
**caractérisé en ce que**,
en fonction des différentes définitions des hétérocyles indiqués R² et R³ ci-dessus,
[A] on transforme des composés de formule générale (II) 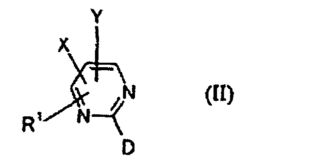 dans laquelle
R¹, X et Y ont la définition indiquée ci-dessus,
et
D représente des restes de formules 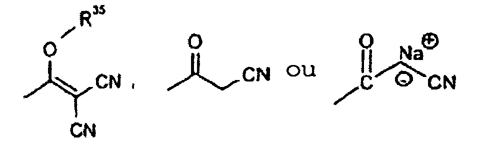 dans lesquels,
R³⁵ est un reste alkyle en C₁ à C₄,
par réaction avec des composés de formule générale (III)
A-CH₂-NH-NH₂ (III)
dans laquelle
A a la définition indiquée ci-dessus,
dans des solvants inertes, éventuellement en présence d'une base, en les composés de formule générale (IV) ou (IVa) 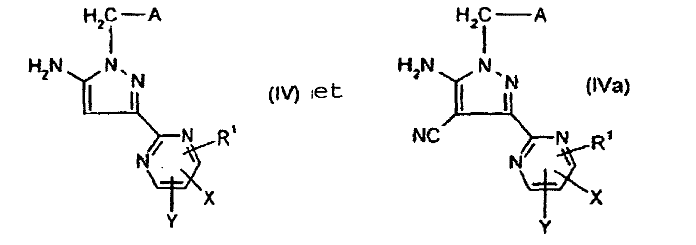 dans laquelle
A, X, Y et R¹ ont la définition indiquée ci-dessus,
et dans le cas des composés de formule générale (IVa), on les cyclise ensuite avec des acides carboxyliques, des nitriles, des formamides ou des sels de guanidium, et dans le cas des composés de formule générale (IV), on les cyclise avec des dérivés 1,3-dicarbonyliques, leurs sels, leurs tautomères, leurs énol-éthers ou leurs énamines, en présence d'acides et le cas échéant sous l'action de micro-ondes,
ou bien
[B] on fait réagir des composés de formule générale (VII) 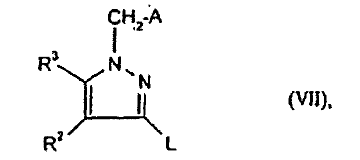 dans laquelle
A¹, R² et R³ ont la définition indiquée ci-dessus, et
L représente un reste de formule -SnR³⁶R³⁷R³⁸, ZnR³⁹, l'iode, le brome ou le radical triflate,
où
R³⁶, R³⁷ et R³⁸ sont identiques ou différents et représentent un reste alkyle linéaire ou ramifié ayant jusqu'à 4 atomes de carbone,
et
R³⁹ représente un halogène,
avec des composés de formule générale (VIII) 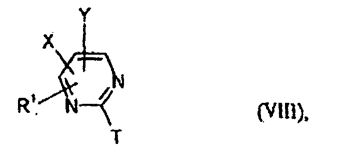 dans laquelle
X, Y et R¹ ont la définition indiquée ci-dessus et
au cas où L représente SnR³⁶R³⁷R³⁸ ou ZnR³⁹,
T est un radical triflate ou un halogène, avantageusement le brome,
et
au cas où L représente l'iode, le brome ou le radical triflate
T est un reste de formule SnR^{36'}R^{37'}R^{38'}, ZnR^{39'} ou BR⁴⁰R⁴¹,
où
R^{36'}, R^{37'}, R^{38'} et R^{39'} ont la définition indiquée ci-dessus pour R³⁶, R³⁷, R³⁸ et R³⁹ et y sont identiques ou en sont différents,
R⁴⁰ et R⁴¹ sont identiques ou différents et représentent un groupe hydroxy, un reste aryloxy ayant 6 à 10 atomes de carbone ou un reste alkyle ou alkoxy linéaire ou ramifié ayant chacun jusqu'à 5 atomes de carbone, ou forment ensemble un noyau carbocyclique pentagonal ou hexagonal,
dans une réaction catalysée par le palladium dans des solvants inertes, le cas échéant en présence d'une base,
ou bien
[C] on fait réagir des amidines de formule générale (IX) 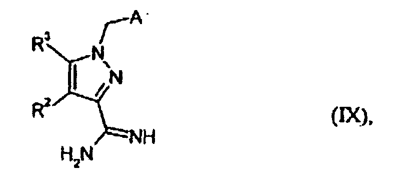 dans laquelle
A, R² et R³ ont la définition indiquée ci-dessus, par exemple avec des composés de formule générale (X), (Xa), (Xb) ou (Xc) 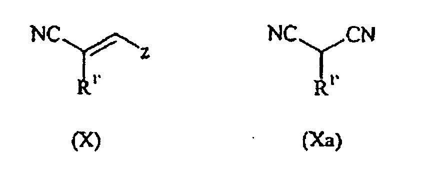 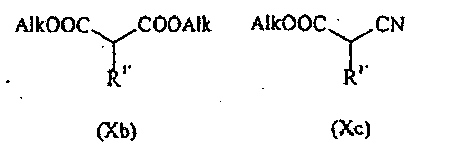 formules dans lesquelles
R^{1'} représente le reste cycloalkyle éventuellement substitué indiqué en R¹ ci-dessus,
Alk désigne un reste alkyle linéaire ou ramifié ayant jusqu'à 8 atomes de carbone, avantageusement jusqu'à 4 atomes de carbone,
et
Z est un groupe NH₂, un groupe monoalkylamino ayant jusqu'à 7 atomes de carbone, un groupe dialkylamino ayant jusqu'à 7 atomes de carbone, un reste pipéridino ou morpholino lié par l'intermédiaire de l'azote, un groupe hydroxyle, un reste alkoxy ayant jusqu'à 7 atomes de carbone, un reste acyloxy ayant jusqu'à 7 atomes de carbone ou un reste aroyloxy ayant 6 à 10 atomes de carbone,
et on fait varier ou on introduit le cas échéant les substituants indiqués en X, Y, R¹, R², R³ et/ou A selon des modes opératoires usuels, avantageusement par acylation et par dérivatisation de groupes amino libres, chloration, hydrogénation catalytique, réduction, oxydation, élimination de groupes protecteurs et/ou substitution nucléophile.

4. Amidines de formule générale (IX) 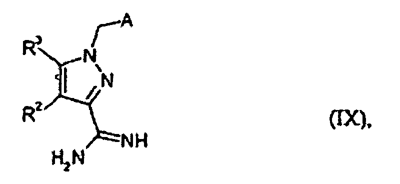 dans laquelle
R², R³ et A sont tels que définis dans la revendication 1, et leurs formes isomères et leurs sels.

5. Médicament contenant au moins un composé de formule générale (I) suivant la revendication 1.

6. Procédé de préparation de médicaments, **caractérisé en ce qu'**on fait prendre une forme convenant pour l'administration à au moins un composé de formule (I) selon la revendication 1, éventuellement avec des substances auxiliaires et des additifs usuels.

7. Médicament contenant au moins un composé de formule générale (I) suivant la revendication 1 en association avec des nitrates organiques ou des donneurs de NO.

8. Médicament contenant au moins un composé de formule générale (I) suivant la revendication 1 en association avec des composés qui inhibent la dégradation du guanosine-monophosphate cyclique (GMPc).

9. Utilisation de composés de formule générale (I) suivant la revendication 1 pour la préparation de médicaments.

10. Utilisation de composés de formule générale (I) suivant la revendication 1 pour la préparation de médicaments destinés au traitement de maladies cardio-vasculaires.

11. Utilisation de composés de formule générale (I) suivant la revendication 1 pour la préparation de médicaments destinés au traitement de l'hypertonie.

12. Utilisation de composés de formule générale (I) suivant la revendication 1 pour la préparation de médicaments destinés au traitement de maladies thromboemboliques et d'ischémie.

13. Utilisation de composés de formule générale (I) suivant la revendication 1 pour la préparation de médicaments destinés au traitement d'un dysfonctionnement sexuel.

14. Utilisation de composés de formule générale (I) suivant la revendication 1 pour la préparation de médicaments possédant des propriétés anti-inflammatoires.

15. Utilisation suivant l'une des revendications 9 à 14, dans laquelle les composés de formule générale (I) selon la revendication 1 sont utilisés en association avec des nitrates organiques ou des donneurs de NO ou en association avec des composés, qui inhibent la dégradation du guanosine-monophosphate cyclique (GMPc).
